# EUROPEAN PATENT APPLICATION

(11) **EP 4 834 931 A1**
(43) Date of publication of application: **04.11.2026**
(21) Application number: 24910457.1
(22) Date of filing: 25.11.2024
(51) Int. Cl.: C07K 16/28, C07K 14/725, A61P 35/00, A61K 39/00

(54) **ANTIBODY TARGETING LY6G6D AND USE THEREOF**

(30) Priority: 26.12.2023 CN 202311797447
(71) Applicant: Sunshine Lake Pharma Co., Ltd., Dongguan, Guangdong 523000 (CN)
(72) Inventor: ZHANG, Kuo, Dongguan, Guangdong 523871 (CN); XIAO, Lin, Dongguan, Guangdong 523871 (CN); REN, Zhiheng, Dongguan, Guangdong 523871 (CN); WAN, Dan, Dongguan, Guangdong 523871 (CN); CHEN, Miaojun, Dongguan, Guangdong 523871 (CN); LIN, Shushan, Dongguan, Guangdong 523871 (CN); LI, Xiang, Dongguan, Guangdong 523871 (CN); LI, Lijia, Dongguan, Guangdong 523871 (CN); PENG, Ju, Dongguan, Guangdong 523871 (CN); LI, Xiaoping, Dongguan, Guangdong 523871 (CN)
(74) Representative: Hoffmann Eitle
(86) International application number: PCT/CN2024/134146
(87) International publication number: WO 2025/139523

(57) **Abstract**

Provided herein are an antibody targeting Ly6G6D and a use thereof. Specifically, provided herein are an anti-Ly6G6D (Lymphocyte Antigen 6 Complex, Locus G61) antibody and antibody fragment, and further provided is a use of the antibody or antibody fragment in preparation of a medicament for treating an Ly6G6D-positive cancer.

## Description

### FIELD OF THE INVENTION

The present invention belongs to the field of antibody engineering. Specifically, the present invention relates to an antibody, and in particular to an antibody targeting Ly6G6D and a use thereof for preparing a medicament

### BACKGROUND ART

Colorectal cancer is a malignant tumor arising from the mucosa, epithelium, and glands of the colon and rectum, and its incidence ranks second only to gastric cancer and esophageal cancer among malignant tumors of the digestive tract. In recent years, due to changes in dietary habits and structure as well as population aging, the morbidity and mortality of colorectal cancer in China have shown an increasing trend.

Lymphocyte antigen 6 family member G6D (Ly6G6D) is a leukocyte antigen cluster located in the major histocompatibility complex (MHC) class III region on chromosome 6, encoding a protein of 133 amino acid residues with a molecular weight of approximately 13.7 kDa. Like most family members, Ly6G6D is attached to the cell membrane via a glycosylphosphatidylinositol (GPI) anchor. Multiple studies have shown that lymphocyte antigen 6 family members play important roles in regulating immune, nervous system, and complement functions. Especially in cancer, many members of the lymphocyte antigen 6 family play critical roles in many types of cancer, such as gastric cancer, cervical cancer, breast cancer, ovarian cancer, lung cancer, and bladder cancer. Therefore, the lymphocyte antigen 6 gene family may play an important role in clinical practice, not only as a marker of disease prognosis but also as an important target for the development of new drugs. In-depth research on their biological functions will be of great significance for elucidating the functions of the lymphocyte antigen 6 family, analyzing protein interactions in their structures, exploring disease pathogenesis, and discovering new therapeutic targets. As a new member of the lymphocyte antigen 6 gene family, Ly6G6D has been successively reported to play an important role in the development of colorectal cancer and other diseases. Currently, a bispecific antibody drug targeting Ly6G6D has been used for the treatment of colorectal cancer, suggesting that Ly6G6D is expected to become a potential target for antibody-based therapy.

### SUMMARY OF THE INVENTION

Provided herein is a fully human anti-Ly6G6D antibody, this antibody has high affinity for human recombinant Ly6G6D protein, and compared to commonly used rabbit-derived or mouse-derived anti-Ly6G6D antibodies, it exhibits better low immunogenicity and safety, and even superior efficacy, demonstrating good application prospects.

Specifically, in one aspect, provided herein is an anti-Ly6G6D antibody or antigen-binding fragment, the antibody or antigen-binding fragment binds to Ly6G6D, and the antibody comprises a heavy chain and a light chain, wherein, the heavy chain comprises a heavy chain variable region, and the heavy chain variable region comprises complementarity determining regions: H-CDR1, H-CDR2 and H-CDR3; wherein,
(1) H-CDR1 has the amino acid sequence shown in SEQ ID NO: 1, SEQ ID NO: 7, SEQ ID NO: 13, SEQ ID NO: 18, SEQ ID NO: 22, SEQ ID NO: 28, SEQ ID NO: 34, SEQ ID NO: 42, SEQ ID NO: 46, SEQ ID NO: 52, SEQ ID NO: 56, SEQ ID NO: 61 or SEQ ID NO: 71; and
(2) H-CDR2 has the amino acid sequence shown in SEQ ID NO: 2, SEQ ID NO: 8, SEQ ID NO: 14, SEQ ID NO: 19, SEQ ID NO: 23, SEQ ID NO: 29, SEQ ID NO: 35, SEQ ID NO: 43, SEQ ID NO: 47, SEQ ID NO: 53, SEQ ID NO: 57 or SEQ ID NO: 72; and
(3) H-CDR3 has the amino acid sequence shown in SEQ ID NO: 3, SEQ ID NO: 9, SEQ ID NO: 15, SEQ ID NO: 20, SEQ ID NO: 24, SEQ ID NO: 30, SEQ ID NO: 36, SEQ ID NO: 40, SEQ ID NO: 44, SEQ ID NO: 48, SEQ ID NO: 54, SEQ ID NO: 58, SEQ ID NO: 62, SEQ ID NO: 66 or SEQ ID NO: 73; and
wherein, the light chain comprises a light chain variable region, the light chain variable region comprises complementarity determining regions: L-CDR1, L-CDR2 and L-CDR3; wherein,
(1) L-CDR1 has an amino acid sequence shown in SEQ ID NO: 4, SEQ ID NO: 10, SEQ ID NO: 25, SEQ ID NO: 31, SEQ ID NO: 37, SEQ ID NO: 49, SEQ ID NO: 59, SEQ ID NO: 63, SEQ ID NO: 68 or SEQ ID NO: 74; and
(2) L-CDR2 has the amino acid sequence shown in SEQ ID NO: 5, SEQ ID NO: 11, SEQ ID NO: 16, SEQ ID NO: 26, SEQ ID NO: 32, SEQ ID NO: 38, SEQ ID NO: 50, SEQ ID NO: 60, SEQ ID NO: 64, SEQ ID NO: 67, SEQ ID NO: 69 or SEQ ID NO: 75; and
(3) L-CDR3 has the amino acid sequence shown in SEQ ID NO: 6, SEQ ID NO: 12, SEQ ID NO: 17, SEQ ID NO: 21, SEQ ID NO: 27, SEQ ID NO: 33, SEQ ID NO: 39, SEQ ID NO: 41, SEQ ID NO: 45, SEQ ID NO: 51, SEQ ID NO: 55, SEQ ID NO: 65, SEQ ID NO: 70 or SEQ ID NO: 76.

In some embodiments, the heavy chain variable region of the present invention comprises the complementarity determining regions: H-CDR1, H-CDR2 and H-CDR3; wherein,
(1) H-CDR1 has the amino acid sequence shown in SEQ ID NO:1, H-CDR2 has the amino acid sequence shown in SEQ ID NO:2 and H-CDR3 has the amino acid sequence shown in SEQ ID NO:3; or
(2) H-CDR1 has the amino acid sequence shown in SEQ ID NO:7, H-CDR2 has the amino acid sequence shown in SEQ ID NO:8 and H-CDR3 has the amino acid sequence shown in SEQ ID NO:9; or
(3) H-CDR1 has the amino acid sequence shown in SEQ ID NO:13, H-CDR2 has the amino acid sequence shown in SEQ ID NO:14 and H-CDR3 has the amino acid sequence shown in SEQ ID NO:15; or
(4) H-CDR1 has the amino acid sequence shown in SEQ ID NO:18, H-CDR2 has the amino acid sequence shown in SEQ ID NO:19 and H-CDR3 has the amino acid sequence shown in SEQ ID NO:20; or
(5) H-CDR1 has the amino acid sequence shown in SEQ ID NO:22, H-CDR2 has the amino acid sequence shown in SEQ ID NO:23 and H-CDR3 has the amino acid sequence shown in SEQ ID NO:24; or
(6) H-CDR1 has the amino acid sequence shown in SEQ ID NO:28, H-CDR2 has the amino acid sequence shown in SEQ ID NO:29 and H-CDR3 has the amino acid sequence shown in SEQ ID NO:30; or
(7) H-CDR1 has the amino acid sequence shown in SEQ ID NO:34, H-CDR2 has the amino acid sequence shown in SEQ ID NO:35 and H-CDR3 has the amino acid sequence shown in SEQ ID NO:36; or
(8) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:40; or
(9) H-CDR1 has the amino acid sequence shown in SEQ ID NO:42, H-CDR2 has the amino acid sequence shown in SEQ ID NO:43 and H-CDR3 has the amino acid sequence shown in SEQ ID NO:44; or
(10) H-CDR1 has the amino acid sequence shown in SEQ ID NO:46, H-CDR2 has the amino acid sequence shown in SEQ ID NO:47 and H-CDR3 has the amino acid sequence shown in SEQ ID NO:48; or
(11) H-CDR1 has the amino acid sequence shown in SEQ ID NO:52, H-CDR2 has the amino acid sequence shown in SEQ ID NO:53 and H-CDR3 has the amino acid sequence shown in SEQ ID NO:54; or
(12) H-CDR1 has the amino acid sequence shown in SEQ ID NO:56, H-CDR2 has the amino acid sequence shown in SEQ ID NO:57 and H-CDR3 has the amino acid sequence shown in SEQ ID NO:58; or
(13) H-CDR1 has the amino acid sequence shown in SEQ ID NO:61, H-CDR2 has the amino acid sequence shown in SEQ ID NO:29 and H-CDR3 has the amino acid sequence shown in SEQ ID NO:62; or
(14) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:66; or
(15) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:62; or
(16) H-CDR1 has the amino acid sequence shown in SEQ ID NO:71, H-CDR2 has the amino acid sequence shown in SEQ ID NO:72 and H-CDR3 has the amino acid sequence shown in SEQ ID NO: 73.

In some embodiments, the light chain variable region of the present invention comprises the complementarity determining regions: L-CDR1, L-CDR2 and L-CDR3; wherein,
(1) L-CDR1 has the amino acid sequence shown in SEQ ID NO:4, L-CDR2 has the amino acid sequence shown in SEQ ID NO:5 and L-CDR3 has the amino acid sequence shown in SEQ ID NO:6; or
(2) L-CDR1 has the amino acid sequence shown in SEQ ID NO:10, L-CDR2 has the amino acid sequence shown in SEQ ID NO:11 and L-CDR3 has the amino acid sequence shown in SEQ ID NO:12; or
(3) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:17; or
(4) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:21; or
(5) L-CDR1 has the amino acid sequence shown in SEQ ID NO:25, L-CDR2 has the amino acid sequence shown in SEQ ID NO:26 and L-CDR3 has the amino acid sequence shown in SEQ ID NO:27; or
(6) L-CDR1 has the amino acid sequence shown in SEQ ID NO:31, L-CDR2 has the amino acid sequence shown in SEQ ID NO:32 and L-CDR3 has the amino acid sequence shown in SEQ ID NO:33; or
(7) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:39; or
(8) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:41; or
(9) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:45; or
(10) L-CDR1 has the amino acid sequence shown in SEQ ID NO:49, L-CDR2 has the amino acid sequence shown in SEQ ID NO:50 and L-CDR3 has the amino acid sequence shown in SEQ ID NO:51; or
(11) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:55; or
(12) L-CDR1 has the amino acid sequence shown in SEQ ID NO:59, L-CDR2 has the amino acid sequence shown in SEQ ID NO:60 and L-CDR3 has the amino acid sequence shown in SEQ ID NO:51; or
(13) L-CDR1 has the amino acid sequence shown in SEQ ID NO:63, L-CDR2 has the amino acid sequence shown in SEQ ID NO:64 and L-CDR3 has the amino acid sequence shown in SEQ ID NO:65; or
(14) L-CDR1 has the amino acid sequence shown in SEQ ID NO:37, L-CDR2 has the amino acid sequence shown in SEQ ID NO:67 and L-CDR3 has the amino acid sequence shown in SEQ ID NO:41; or
(15) L-CDR1 has the amino acid sequence shown in SEQ ID NO:68, L-CDR2 has the amino acid sequence shown in SEQ ID NO:69 and L-CDR3 has the amino acid sequence shown in SEQ ID NO:70; or
(16) L-CDR1 has the amino acid sequence shown in SEQ ID NO:74, L-CDR2 has the amino acid sequence shown in SEQ ID NO:75 and L-CDR3 has the amino acid sequence shown in SEQ ID NO: 76.

In some embodiments, the heavy chain variable region of the present invention comprises the complementarity determining regions: H-CDR1, H-CDR2 and H-CDR3; and the light chain variable region of the present invention comprises the complementarity determining regions: L-CDR1, L-CDR2 and L-CDR3; wherein,
(1) H-CDR1 has the amino acid sequence shown in SEQ ID NO:1, H-CDR2 has the amino acid sequence shown in SEQ ID NO:2 and H-CDR3 has the amino acid sequence shown in SEQ ID NO:3; and
   L-CDR1 has the amino acid sequence shown in SEQ ID NO:4, L-CDR2 has the amino acid sequence shown in SEQ ID NO:5 and L-CDR3 has the amino acid sequence shown in SEQ ID NO:6; or
(2) H-CDR1 has the amino acid sequence shown in SEQ ID NO:7, H-CDR2 has the amino acid sequence shown in SEQ ID NO:8 and H-CDR3 has the amino acid sequence shown in SEQ ID NO:9; and
   L-CDR1 has the amino acid sequence shown in SEQ ID NO:10, L-CDR2 has the amino acid sequence shown in SEQ ID NO:11 and L-CDR3 has the amino acid sequence shown in SEQ ID NO:12; or
(3) H-CDR1 has the amino acid sequence shown in SEQ ID NO:13, H-CDR2 has the amino acid sequence shown in SEQ ID NO:14 and H-CDR3 has the amino acid sequence shown in SEQ ID NO:15; and
   L-CDR1 has the amino acid sequence shown in SEQ ID NO:4, L-CDR2 has the amino acid sequence shown in SEQ ID NO:16 and L-CDR3 has the amino acid sequence shown in SEQ ID NO:17; or
(4) H-CDR1 has the amino acid sequence shown in SEQ ID NO:18, H-CDR2 has the amino acid sequence shown in SEQ ID NO:19 and H-CDR3 has the amino acid sequence shown in SEQ ID NO:20; and
   L-CDR1 has the amino acid sequence shown in SEQ ID NO:4, L-CDR2 has the amino acid sequence shown in SEQ ID NO:16 and L-CDR3 has the amino acid sequence shown in SEQ ID NO:21; or
(5) H-CDR1 has the amino acid sequence shown in SEQ ID NO:22, H-CDR2 has the amino acid sequence shown in SEQ ID NO:23 and H-CDR3 has the amino acid sequence shown in SEQ ID NO:24; and
   L-CDR1 has the amino acid sequence shown in SEQ ID NO:25, L-CDR2 has the amino acid sequence shown in SEQ ID NO:26 and L-CDR3 has the amino acid sequence shown in SEQ ID NO:27; or
(6) H-CDR1 has the amino acid sequence shown in SEQ ID NO:28, H-CDR2 has the amino acid sequence shown in SEQ ID NO:29 and H-CDR3 has the amino acid sequence shown in SEQ ID NO: 30; and
   L-CDR1 has the amino acid sequence shown in SEQ ID NO:31, L-CDR2 has the amino acid sequence shown in SEQ ID NO:32 and L-CDR3 has the amino acid sequence shown in SEQ ID NO:33; or
(7) H-CDR1 has the amino acid sequence shown in SEQ ID NO:34, H-CDR2 has the amino acid sequence shown in SEQ ID NO:35 and H-CDR3 has the amino acid sequence shown in SEQ ID NO:36; and
   L-CDR1 has the amino acid sequence shown in SEQ ID NO:37, L-CDR2 has the amino acid sequence shown in SEQ ID NO:38 and L-CDR3 has the amino acid sequence shown in SEQ ID NO:39; or
(8) H-CDR1 has the amino acid sequence shown in SEQ ID NO:18, H-CDR2 has the amino acid sequence shown in SEQ ID NO:29 and H-CDR3 has the amino acid sequence shown in SEQ ID NO: 40; and
   L-CDR1 has the amino acid sequence shown in SEQ ID NO:37, L-CDR2 has the amino acid sequence shown in SEQ ID NO:38 and L-CDR3 has the amino acid sequence shown in SEQ ID NO:41; or
(9) H-CDR1 has the amino acid sequence shown in SEQ ID NO:42, H-CDR2 has the amino acid sequence shown in SEQ ID NO:43 and H-CDR3 has the amino acid sequence shown in SEQ ID NO:44; and
   L-CDR1 has the amino acid sequence shown in SEQ ID NO:37, L-CDR2 has the amino acid sequence shown in SEQ ID NO:38 and L-CDR3 has the amino acid sequence shown in SEQ ID NO:45; or
(10) H-CDR1 has the amino acid sequence shown in SEQ ID NO:46, H-CDR2 has the amino acid sequence shown in SEQ ID NO:47 and H-CDR3 has the amino acid sequence shown in SEQ ID NO:48; and
   L-CDR1 has the amino acid sequence shown in SEQ ID NO:49, L-CDR2 has the amino acid sequence shown in SEQ ID NO:50 and L-CDR3 has the amino acid sequence shown in SEQ ID NO:51; or
(11) H-CDR1 has the amino acid sequence shown in SEQ ID NO:52, H-CDR2 has the amino acid sequence shown in SEQ ID NO:53 and H-CDR3 has the amino acid sequence shown in SEQ ID NO:54; and
   L-CDR1 has the amino acid sequence shown in SEQ ID NO:37, L-CDR2 has the amino acid sequence shown in SEQ ID NO:38 and L-CDR3 has the amino acid sequence shown in SEQ ID NO:55; or
(12) H-CDR1 has the amino acid sequence shown in SEQ ID NO:56, H-CDR2 has the amino acid sequence shown in SEQ ID NO:57 and H-CDR3 has the amino acid sequence shown in SEQ ID NO:58; and
   L-CDR1 has the amino acid sequence shown in SEQ ID NO:59, L-CDR2 has the amino acid sequence shown in SEQ ID NO:60 and L-CDR3 has the amino acid sequence shown in SEQ ID NO:51; or
(13) H-CDR1 has the amino acid sequence shown in SEQ ID NO:61, H-CDR2 has the amino acid sequence shown in SEQ ID NO:29 and H-CDR3 has the amino acid sequence shown in SEQ ID NO: 62; and
   L-CDR1 has the amino acid sequence shown in SEQ ID NO:63, L-CDR2 has the amino acid sequence shown in SEQ ID NO:64 and L-CDR3 has the amino acid sequence shown in SEQ ID NO:65; or
(14) H-CDR1 has the amino acid sequence shown in SEQ ID NO:18, H-CDR2 has the amino acid sequence shown in SEQ ID NO:29 and H-CDR3 has the amino acid sequence shown in SEQ ID NO: 66; and
   L-CDR1 has the amino acid sequence shown in SEQ ID NO:37, L-CDR2 has the amino acid sequence shown in SEQ ID NO:67 and L-CDR3 has the amino acid sequence shown in SEQ ID NO:41; or
(15) H-CDR1 has the amino acid sequence shown in SEQ ID NO:18, H-CDR2 has the amino acid sequence shown in SEQ ID NO:29 and H-CDR3 has the amino acid sequence shown in SEQ ID NO: 62; and
   L-CDR1 has the amino acid sequence shown in SEQ ID NO:68, L-CDR2 has the amino acid sequence shown in SEQ ID NO:69 and L-CDR3 has the amino acid sequence shown in SEQ ID NO:70; or
(16) H-CDR1 has the amino acid sequence shown in SEQ ID NO:71, H-CDR2 has the amino acid sequence shown in SEQ ID NO:72 and H-CDR3 has the amino acid sequence shown in SEQ ID NO: 73; and
   L-CDR1 has the amino acid sequence shown in SEQ ID NO:74, L-CDR2 has the amino acid sequence shown in SEQ ID NO:75 and L-CDR3 has the amino acid sequence shown in SEQ ID NO: 76.

In some embodiments, the CDR sequences of the present invention are defined with reference to the Kabat numbering system.
NYAMH (SEQ ID NO: 1).
VISYDGSKKYYADSVKG (SEQ ID NO: 2).
PAGSTSGFDY (SEQ ID NO: 3).
TGTSSDVGGYNYVS (SEQ ID NO: 4).
DVSNRPS (SEQ ID NO: 5).
SSYTSSSTLVV (SEQ ID NO: 6).
DYGMS (SEQ ID NO: 7).
GINWNGGSTGYADSVKG (SEQ ID NO: 8).
SSVKHY (SEQ ID NO: 9).
SGSSSNIGTNTVN (SEQ ID NO: 10).
RNDLRPS (SEQ ID NO: 11).
AAWDDSLNGVV (SEQ ID NO: 12).
NYGIT (SEQ ID NO: 13).
WISPYNGHTIYAQKVQG (SEQ ID NO: 14).
GLNSRLQY (SEQ ID NO: 15).
DVSKRPS (SEQ ID NO: 16).
SSYTSSSTDV (SEQ ID NO: 17).
GYYMH (SEQ ID NO: 18).
WINPVSGGTNYAQKFQG (SEQ ID NO: 19).
DPFDI (SEQ ID NO: 20).
SSYTSRSTPGYV (SEQ ID NO: 21).
NYGFS (SEQ ID NO: 22).
WISSYNGNTKYAQKFRG (SEQ ID NO: 23).
GGDWPADY (SEQ ID NO: 24).
GLSSGSVSNNYYPS (SEQ ID NO: 25).
STNTRSS (SEQ ID NO: 26).
VVYMGSGIWV (SEQ ID NO: 27).
SYGIS (SEQ ID NO: 28).
WINPNSGGTNYAQKFQG (SEQ ID NO: 29).
AGDLRDYYYYGMDV (SEQ ID NO: 30).
TGSSSNIGAGYDVH (SEQ ID NO: 31).
GNSNRPS (SEQ ID NO: 32).
QSYDSSLSGWV (SEQ ID NO: 33).
RYPIH (SEQ ID NO: 34).
WINTNTGNPTYAQGFTG (SEQ ID NO: 35).
SGELGGALDI (SEQ ID NO: 36).
RSSQSLLHSNGYNYLD (SEQ ID NO: 37).
LGSNRAS (SEQ ID NO: 38).
MQPLQTPLT (SEQ ID NO: 39).
VGHYYYGMDV (SEQ ID NO: 40).
MQALQTPPT (SEQ ID NO: 41).
NHFIH (SEQ ID NO: 42).
WISANSGNTDYAQKFQG (SEQ ID NO: 43).
DLATVDGFDI (SEQ ID NO: 44).
MQALQTPLT (SEQ ID NO: 45).
SYSVS (SEQ ID NO: 46).
GIIPTFGRTNYAQKFQG (SEQ ID NO: 47).
VRKPYYGMDV (SEQ ID NO: 48).
RASQSIGRFLN (SEQ ID NO: 49).
GASRLQG (SEQ ID NO: 50).
QQSYSTPPT (SEQ ID NO: 51).
SYGIN (SEQ ID NO: 52).
WINTKTGNPTYVQGFTG (SEQ ID NO: 53).
DRNPYGMDV (SEQ ID NO: 54).
MQPLQALYT (SEQ ID NO: 55).
SYAIS (SEQ ID NO: 56).
GIIPIFGTANYAQKFQG (SEQ ID NO: 57).
TGGDYYYYGMDV (SEQ ID NO: 58).
RASQTISTYLN (SEQ ID NO: 59).
GASSLQT (SEQ ID NO: 60).
DYFLH (SEQ ID NO: 61).
DPFDY (SEQ ID NO: 62).
RSSQSLVHSDGNTYLS (SEQ ID NO: 63).
KISNRFS (SEQ ID NO: 64).
MQATQFPIT (SEQ ID NO: 65).
VGGSYYGGFDY (SEQ ID NO: 66).
LSSNRAS (SEQ ID NO: 67).
RSSQSLVYSDGNTYLS (SEQ ID NO: 68).
KVSNRFS (SEQ ID NO: 69).
MQATQYPLA (SEQ ID NO: 70).
NYWIG (SEQ ID NO: 71).
IIYPGDSDTRYSPSFQG (SEQ ID NO: 72).
PQGAFDI (SEQ ID NO: 73).
RSSQSLVYSDGNTYLN (SEQ ID NO: 74).
KVSNRDS (SEQ ID NO: 75).
MQGTHWPLT (SEQ ID NO: 76).

In some embodiments, the antibody of the present invention comprises at least one of a heavy chain framework region sequence and a light chain framework region sequence, at least a part of at least one of the heavy chain framework region sequence and the light chain framework region sequence is derived from at least one of a murine antibody, a human antibody, a primate antibody or a mutant thereof.

In some embodiments, the antibody heavy chain variable region of the present invention comprises an amino acid sequence having at least 85%, at least 90%, at least 95%, or at least 99% sequence identity to any one of SEQ ID NO:77-92.

In some embodiments, the antibody heavy chain variable region comprises an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to any one of SEQ ID NO:77-92.

In some embodiments, the antibody light chain variable region of the present invention comprises an amino acid sequence having at least 85%, at least 90%, at least 95%, or at least 99% sequence identity to any one of SEQ ID NO:93-108.

In some embodiments, the antibody light chain variable region of the present invention comprises an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to any one of SEQ ID NO:93-108.

In some embodiments, the antibody heavy chain variable region of the present invention comprises an amino acid sequence shown in SEQ ID NO:77 and the light chain variable region comprises an amino acid sequence shown in SEQ ID NO:93.

In the present invention, the antibody clone comprising the heavy chain variable region shown in SEQ ID NO:77 and the light chain variable region shown in SEQ ID NO:93 is designated as: 2E7.

In some embodiments, the antibody heavy chain variable region of the present invention comprises an amino acid sequence shown in SEQ ID NO:78 and the light chain variable region comprises an amino acid sequence shown in SEQ ID NO:94.

In the present invention, the antibody clone comprising the heavy chain variable region shown in SEQ ID NO:78 and the light chain variable region shown in SEQ ID NO:94 is designated as: 2G10.

The antibody heavy chain variable region of the present invention comprises an amino acid sequence shown in SEQ ID NO:79 and the light chain variable region comprises an amino acid sequence shown in SEQ ID NO:95.

In the present invention, the antibody clone comprising the heavy chain variable region shown in SEQ ID NO:79 and the light chain variable region shown in SEQ ID NO:95 is designated as: 5C12.

The antibody heavy chain variable region of the present invention comprises an amino acid sequence shown in SEQ ID NO:80 and the light chain variable region comprises an amino acid sequence shown in SEQ ID NO:96.

In the present invention, the antibody clone comprising the heavy chain variable region shown in SEQ ID NO:80 and the light chain variable region shown in SEQ ID NO:96 is designated as: 6C1.

The antibody heavy chain variable region of the present invention comprises an amino acid sequence shown in SEQ ID NO:81 and the light chain variable region comprises an amino acid sequence shown in SEQ ID NO:97.

In the present invention, the antibody clone comprising the heavy chain variable region shown in SEQ ID NO:81 and the light chain variable region shown in SEQ ID NO:97 is designated as: 7B1.

The antibody heavy chain variable region of the present invention comprises an amino acid sequence shown in SEQ ID NO:82 and the light chain variable region comprises an amino acid sequence shown in SEQ ID NO:98.

In the present invention, the antibody clone comprising the heavy chain variable region shown in SEQ ID NO:82 and the light chain variable region shown in SEQ ID NO:98 is designated as: 12A1.

The antibody heavy chain variable region of the present invention comprises an amino acid sequence shown in SEQ ID NO:83 and the light chain variable region comprises an amino acid sequence shown in SEQ ID NO:99.

In the present invention, the antibody clone comprising the heavy chain variable region shown in SEQ ID NO:83 and the light chain variable region shown in SEQ ID NO:99 is designated as: 13A10.

The antibody heavy chain variable region of the present invention comprises an amino acid sequence shown in SEQ ID NO:84 and the light chain variable region comprises an amino acid sequence shown in SEQ ID NO:100.

In the present invention, the antibody clone comprising the heavy chain variable region shown in SEQ ID NO:84 and the light chain variable region shown in SEQ ID NO:100 is designated as: 14A2.

The antibody heavy chain variable region of the present invention comprises an amino acid sequence shown in SEQ ID NO:85 and the light chain variable region comprises an amino acid sequence shown in SEQ ID NO:101.

In the present invention, the antibody clone comprising the heavy chain variable region shown in SEQ ID NO:85 and the light chain variable region shown in SEQ ID NO:101 is designated as: 14B5.

The antibody heavy chain variable region of the present invention comprises an amino acid sequence shown in SEQ ID NO:86 and the light chain variable region comprises an amino acid sequence shown in SEQ ID NO:102.

In the present invention, the antibody clone comprising the heavy chain variable region shown in SEQ ID NO:86 and the light chain variable region shown in SEQ ID NO:102 is designated as: 14G2.

The antibody heavy chain variable region of the present invention comprises an amino acid sequence shown in SEQ ID NO:87 and the light chain variable region comprises an amino acid sequence shown in SEQ ID NO:103.

In the present invention, the antibody clone comprising the heavy chain variable region shown in SEQ ID NO:87 and the light chain variable region shown in SEQ ID NO:103 is designated as: 16H6.

The antibody heavy chain variable region of the present invention comprises an amino acid sequence shown in SEQ ID NO:88 and the light chain variable region comprises an amino acid sequence shown in SEQ ID NO:104.

In the present invention, the antibody clone comprising the heavy chain variable region shown in SEQ ID NO:88 and the light chain variable region shown in SEQ ID NO:104 is designated as: 17A11.

The antibody heavy chain variable region of the present invention comprises an amino acid sequence shown in SEQ ID NO:89 and the light chain variable region comprises an amino acid sequence shown in SEQ ID NO:105.

In the present invention, the antibody clone comprising the heavy chain variable region shown in SEQ ID NO:89 and the light chain variable region shown in SEQ ID NO:105 is designated as: 18A11.

The antibody heavy chain variable region of the present invention comprises an amino acid sequence shown in SEQ ID NO:90 and the light chain variable region comprises an amino acid sequence shown in SEQ ID NO:106.

In the present invention, the antibody clone comprising the heavy chain variable region shown in SEQ ID NO:90 and the light chain variable region shown in SEQ ID NO:106 is designated as: 19A4.

The antibody heavy chain variable region of the present invention comprises an amino acid sequence shown in SEQ ID NO:91 and the light chain variable region comprises an amino acid sequence shown in SEQ ID NO:107.

In the present invention, the antibody clone comprising the heavy chain variable region shown in SEQ ID NO:91 and the light chain variable region shown in SEQ ID NO:107 is designated as: 20E2.

The antibody heavy chain variable region of the present invention comprises an amino acid sequence shown in SEQ ID NO:92 and the light chain variable region comprises an amino acid sequence shown in SEQ ID NO:108.

In the present invention, the antibody clone comprising the heavy chain variable region shown in SEQ ID NO:92 and the light chain variable region shown in SEQ ID NO:108 is designated as: 22A6.

EVHLVESGGGVVQPGRSLRLSCAASGFTFSNYAMHWVRQAPGKGLEWVAVISYDGSK
KYYADSVKGRFTISRDDSKNTLYLQMNSLRAEDTAVYYCAKPAGSTSGFDYWGQGTLVTV
SS (SEQ ID NO: 77).

QSALTQPASVSGSPGQSITISCTGTSSDVGGYNYVSWYQQHPGKAPKLMIYDVSNRPSG
VSNRFSGSKSGNTASLTISGLQAEDEADYYCSSYTSSSTLVVFGGGTKLTVL (SEQ ID NO: 93).
QVQLVQSGGGVVRPGGSLRLSCAASGFTFDDYGMSWVRQAPGKGLEWVSGINWNGG
STGYADSVKGRFTISRDNSKNTLYLQMNSLRAEDTAVYYCATSSVKHYWGQGTLVTVSS
(SEQ ID NO: 78).

QPVLTQPPSASATPGQRVTISCSGSSSNIGTNTVNWYQQVPGTAPKVLIYRNDLRPSGVP
DRFSGSKSGTSASLAISGLQSDDEADYYCAAWDDSLNGVVFGGGTKVTVL (SEQ ID NO:
94).

QVQLVQSGPEMKKPGASVKVACKTSGYIFINYGITWVRQAPGQGLEWMGWISPYNGH
TIYAQKVQGRGTMTTETPTSTAYLDLTNLTPDDTAIYYCATGLNSRLQYWGQGALVTVSS
(SEQ ID NO: 79).

QPVLTQPASVSGSPGQSITISCTGTSSDVGGYNYVSWYQQHPGKAPKLMIYDVSKRPSG
VSNRFSGSKSGNTASLTISGLQAEDEADYYCSSYTSSSTDVFGTGTKVTVL (SEQ ID NO: 95).

EVQLVQSGAEVKKPGASMKVSCKASGYTFTGYYMHWVRQAPGQGLEWMGWINPVS
GGTNYAQKFQGRVTMTRDTSIGTAYMELTGLRSDDTAVYYCAADPFDIWGQGTMVTVSS
(SEQ ID NO: 80).

QSALTQPASVSGSPGQSITISCTGTSSDVGGYNYVSWYQQHPGKAPKLMIYDVSKRPSG
VSNRFSGSKSGNTASLTISGLQAEDEADYYCSSYTSRSTPGYVFGTGTKLTVL (SEQ ID NO:
96).

QVQLVQSGAEVKKPGASVKVSCKASGYTFTNYGFSWVRQAPGQGLEWMGWISSYNG
NTKYAQKFRGRVTMTTDTSTSTGYMELRSLRSDDTAVYYCARGGDWPADYWGQGTLVTV
SS (SEQ ID NO: 81).

QAVVTQEPSFSVSPGGTVTLTCGLSSGSVSNNYYPSWYQQIPGQAPRTLIYSTNTRSSGV
PDRFSGSILGNKAALTITGAQADDEADYYCVVYMGSGIWVFGGGTKLTVL (SEQ ID NO: 97).

QVQLVQSGAEVKKPGASVKVSCKASGYTFTSYGISWVRQAPGQGLEWMGWINPNSGG
TNYAQKFQGRVTMTRDTSISTAYMELSRLRSDDTAVYYCARAGDLRDYYYYGMDVWGQG
TTVTVSS (SEQ ID NO: 82).

QSVLTQPPSVSGAPGQRVTISCTGSSSNIGAGYDVHWYQQLPGTAPKLLIYGNSNRPSGV
PDRFSGSKSGTSASLAITGLQAEDEADYYCQSYDSSLSGWVFGGGTQLTAL (SEQ ID NO:
98).

EVQLVQSGSELKNPGASVKVSCKASGYPFSRYPIHWVRQAPGQGLEWMGWINTNTGN
PTYAQGFTGRFVFSLDTSVSTAYLQISSLKAEDSAVYYCARSGELGGALDIWGQGTTVTVSS
(SEQ ID NO: 83).

EIVLTQSPLSLSVTPGEPASFSCRSSQSLLHSNGYNYLDWYVQKPGQSPQLLIYLGSNRA
SGVPDRFSGSGSGTDFTLKISRVEAEDVGIYYCMQPLQTPLTFGGGTKVEIK (SEQ ID NO:
99).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCARVGHYYYGMDVWGQGTT
VTVSS (SEQ ID NO: 84).

EIVLTQSPLSLPVTPGEPASISCRSSQSLLHSNGYNYLDWYLQKPGQSPQLLIYLGSNRAS
GVPDRFSGSGSGTDFTLKISRVEAEDVGVYYCMQALQTPPTFGQGTKLEIK (SEQ ID NO:
100).

QVQLVQSGAEVKKPGASVKVSCKASGYTFTNHFIHWVRQAPGQGLEWMGWISANSG
NTDYAQKFQGRVTMTTDTSTSTAYMELRSLRYDDTAVYYCARDLATVDGFDIWGQGTMVT
VSS (SEQ ID NO: 85).

DVVMTQSPLSLPVTPGEPASISCRSSQSLLHSNGYNYLDWYLQKPGQSPQLLIYLGSNR
ASGVPDRFSGSGSGTDFTLKISRVEAEDVGVYYCMQALQTPLTFGPGTKVDIK (SEQ ID NO:
101).

EVQLVQSGSEVKKPGSSVKVSCKAPGGTFSSYSVSWVRQAPGQGLEWMGGIIPTFGRT
NYAQKFQGRVTISAHESTGTAYMELSSLRSEDTAVYYCASVRKPYYGMDVWGQGTMVTVS
S (SEQ ID NO: 86).

DIQMTQSPSSLSASVGDRVTITCRASQSIGRFLNWYQQKPGTAPKLLISGASRLQGGIPSR
FSGSGSGTDFTLTISSLQPEDFATYYCQQSYSTPPTFGQGTKVEIK (SEQ ID NO: 102).

QVQLVQSGSELKKPGASVKISCKASGYTLTSYGINWVRQAPGKGLELMGWINTKTGNP
TYVQGFTGQVVFSFDTSVNTAYLQINNLKSDDTAVYFCARDRNPYGMDVWGQGTTVTVSS
(SEQ ID NO: 87).

EIVLTQSPLSLPVTPGEPASISCRSSQSLLHSNGYNYLDWYLQKPGQSPQLLIYLGSNRAS
GVPDRFSGSGSGTDFTLKISRVEAEDVGVYYCMQPLQALYTFGQGTKVEIK (SEQ ID NO:
103).

QVQLVQSGAEVKKPGSSVKVSCKASGGTFSSYAISWVRQAPGQGLEWMGGIIPIFGTAN
YAQKFQGRVTITADESTSTAYMELSSLRSEDTAVYYCARTGGDYYYYGMDVWGQGTTVTV
SS (SEQ ID NO: 88).

DIQMTQSPSSLSASVGDRVTITCRASQTISTYLNWYQQKPGKAPKLLIYGASSLQTGVPS
RFSGSGSGTDFTLTISSLQPEDFATYYCQQSYSTPPTFGQGTKLEIK (SEQ ID NO: 104).

EVQLVESGAEVKKPGASVKVSCKASGYTFTDYFLHWVRQAPGQGLEWMGWINPNSG
GTNYAQKFQGWVTMTRDTSISTAYLELSSLTSDDTAVYYCAVDPFDYWGQGTLVTVSS
(SEQ ID NO: 89).

DVVMTQSPLSSPVTLGQPASISCRSSQSLVHSDGNTYLSWLHQRPGQPPRLLIHKISNRFS
GVPDRFSGSGAGTDFTLKISRVEAEDVGVFYCMQATQFPITFGQGTRLEIK (SEQ ID NO:
105).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCARVGGSYYGGFDYWGQGTL
VTVSS (SEQ ID NO: 90).

EIVLTQSPLSLPVTPGEPASISCRSSQSLLHSNGYNYLDWYLQKPGQSPQLLMYLSSNRA
SGVPDRFSGSGSGRDFTLKISRVEAEDVGLYYCMQALQTPPTFGQGTRLEIK (SEQ ID NO:
106).

QVQLVQSGAEVKKPGASVKVSCKASGYTFTGYYMHWVRQAPGQGLEWMGWINPNS
GGTNYAQKFQGRVTMTRDTSISTAYMELSRLRSDDTAVYYCASDPFDYWGQGTLVTVSS
(SEQ ID NO: 91).

DVVMTQSPLSSPVTLGQPASISCRSSQSLVYSDGNTYLSWLQQRPGQPPRLLIYKVSNRF
SGVPDRFSGSGAGTDFTLTISRVEAEDVGVYYCMQATQYPLAFGGGTKLEIK (SEQ ID NO:
107).

QVQLVQSGAEVKKPGESLKISCKGSGYSFTNYWIGWVRQMPGKGLEWMGIIYPGDSDT
RYSPSFQGQVTISADKSISTAYLQWSSLKASDTAMYYCARPQGAFDIWGQGTMVTVSS

(SEQ ID NO: 92).

EIVMTQTPLSLPVTLGQPASISCRSSQSLVYSDGNTYLNWFQQRPGQSPRRLIYKVSNRD
SGVPDRFSGSGSGTDFTLKISRVEAEDVGVYYCMQGTHWPLTFGQGTKVEIK (SEQ ID NO:
108).

In some embodiments, the antibody of the present invention comprises at least one of a heavy chain constant region and a light chain constant region, at least a part of at least one of the heavy chain constant region and the light chain constant region is derived from at least one of a murine antibody, a human antibody, a primate antibody or a mutant thereof.

In some embodiments, both the antibody heavy chain constant region and the antibody light chain constant region of the present invention are derived from a human IgG antibody or a mutant thereof.

In some embodiments, the antibody heavy chain constant region of the present invention is derived from a human IgG1 or IgG4 antibody or a mutant thereof.

In some embodiments, the antibody light chain constant region of the present invention is derived from a human IgGκ or a IgGλ constant region.

In some embodiments, the antibody heavy chain constant region of the present invention comprises the full-length sequence of the constant region, and the antibody light chain constant region comprises the full-length sequence of the constant region.

In some embodiments, the antibody heavy chain of the present invention comprises heavy chain variable region and constant region; the heavy chain variable region comprises an amino acid sequence having at least 85%, at least 90%, at least 95%, or at least 99% sequence identity to any one of SEQ ID NO:77-92.

In some embodiments, the antibody heavy chain of the present invention comprises heavy chain variable region and constant region; the heavy chain variable region comprises an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to any one of SEQ ID NO:77-92.

In some embodiments, the antibody light chain of the present invention comprises light chain variable region and constant region; the light chain variable region comprises an amino acid sequence having at least 85%, at least 90%, at least 95%, or at least 99% sequence identity to any one of SEQ ID NO:93-108.

In some embodiments, the antibody light chain of the present invention comprises light chain variable region and constant region; the light chain variable region comprises an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to any one of SEQ ID NO:93-108.

In some embodiments, the antibody of the present invention is monoclonal antibody.

In some embodiments, the antibody of the present invention is monoclonal human antibody.

In some embodiments, the antigen-binding fragment of the antibody of the present invention is selected from: Fab, Fab'-SH, Fv and (Fab')2 fragments.

In some embodiments, the antibody of the present invention is a natural intact antibody, a single-chain antibody (scFv), a single-chain Fv-Fc antibody (scFv-Fc), an scFv dimer, or a dimer of an scFv-Fc antibody. Wherein, the dimer includes homodimers and heterodimers.

The single-chain antibody (scFv) as described above comprises a heavy chain variable region having an amino acid sequence shown in any one of SEQ ID NO:77-92 and a light chain variable region having an amino acid sequence shown in any one of SEQ ID NO:93-108, wherein the C-terminus of the heavy chain variable region is connected to the N-terminus of the light chain variable region via a peptide Linker, or the C-terminus of the variable region is connected to the N-terminus of the heavy chain variable region via a peptide Linker . It should be noted that the "peptide Linker" of the single-chain antibody of the present invention is used to connect the heavy chain variable region and the light chain variable region of the antibody, and can be a conventional peptide Linker commonly used for preparing single-chain antibodies, or a peptide Linker modified by researchers. An example of the amino acid sequence of the peptide Linker is: (GnS)m, wherein n is a natural number from 3 to 5, preferably 4, and m is a natural number from 1 to 5, preferably 3.
GGGS (SEQ ID NO: 109).
GGGGS (SEQ ID NO: 110).
GGGGSGGGGS (SEQ ID NO: 111).
GGGGSGGGGSGGGGS (SEQ ID NO: 112).

As a specific example, the single-chain antibody (scFv) of the present invention comprises the amino acid sequence shown in SEQ ID NO:113.

EVHLVESGGGVVQPGRSLRLSCAASGFTFSNYAMHWVRQAPGKGLEWVAVISYDGSK
KYYADSVKGRFTISRDDSKNTLYLQMNSLRAEDTAVYYCAKPAGSTSGFDYWGQGTLVTV

SSGGGGSGGGGSGGGGSQSALTQPASVSGSPGQSITISCTGTSSDVGGYNYVSWYQQHPG
KAPKLMIYDVSNRPSGVSNRFSGSKSGNTASLTISGLQAEDEADYYCSSYTSSSTLVVFGGG
TKLTVL (SEQ ID NO: 113).

In the amino acid sequence shown in SEQ ID NO:113, the bolded portion is the peptide Linker.

The single-chain antibody of the present invention can be constructed with reference to the specific examples described above.

The single-chain Fv-Fc antibody (scFv-Fc) as described above comprises an scFv and an Fc fragment, wherein the Fc fragment refers to a fragment of an antibody heavy chain constant region comprising at least a hinge region, CH2 and CH3 domains; the scFv is as described above, and the scFv is directly connected to the Fc fragment or connected via a peptide Linker (a specific example of the peptide Linker is GGGGSGGGGSGGGGS). In some embodiments, the scFv of the present invention is directly connected to the Fc fragment via the hinge region.

The Fc fragment as described above is derived from, for example, the heavy chain constant region of human IgG1, IgG2, IgG3, IgG4, IgM, IgA1, IgA2, IgD, or IgE; particularly selected from, for example, the heavy chain constant region of human IgG1, IgG2, IgG3, or IgG4, more particularly selected from the heavy chain constant region of human IgG1 or IgG4; and the Fc fragment has one or more amino acid substitutions, deletions, or additions (e.g., up to 20, up to 15, up to 10, up to 5, up to 3, or up to 1 substitution, deletion, or addition) compared to the native sequence from which it is derived.

Specifically, the Fc in the single-chain Fv-Fc antibody (scFv-Fc) of the present invention has the amino acid sequence shown in SEQ ID NO:114.

EPKSADKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEK
TISKAKGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK (SEQ ID NO:
114).

In some embodiments, the antibody of the present invention is a monospecific antibody, a bispecific antibody, or a polyspecific antibody.

In another aspect, provided herein is an isolated nucleic acid molecule, the nucleic acid molecule comprises a nucleotide sequence encoding the antibody or antigen-binding fragment of the present invention.

In some embodiments, the nucleic acid molecule of the present invention is DNA.

In yet another aspect, provided herein is a nucleic acid construct, wherein the nucleic acid construct comprises the nucleic acid molecule of the present invention; further, the nucleic acid construct comprises one or more control sequences operably linked to the nucleic acid molecule, the one or more control sequences directing the production of the antibody or antigen-binding fragment thereof of the present invention in a suitable host cell, wherein the one or more control sequences are selected from: a promoter, an enhancer, a stop signal, a signal peptide, a leader sequence, a transcription terminator, and any combination thereof.

In yet another aspect, provided herein is an expression vector carrying the nucleic acid molecule of the present invention.

In some embodiments, the expression vector of the present invention is selected from the group consisting of: plasmids, cosmids, viruses, minichromosomes, and artificial chromosomes.

In some embodiments, the expression vector of the present invention is a eukaryotic expression vector.

In some embodiments, the eukaryotic expression vector of the present invention is selected from the group consisting of: pCRII, pCR3, pcDNA3.4, pBSII, pET15, pGEX, pEGFP-N1, pETL, pDSR-α, and pFastBacDual.

In yet another aspect, provided herein is a host cell comprising the nucleic acid molecule of the present invention or the expression vector of the present invention.

In some embodiments, the host cell of the present invention is Escherichia coli, yeast, or a eukaryotic cell.

In some embodiments, the host cell of the present invention is a eukaryotic host cell.

In some embodiments, the eukaryotic host cell of the present invention is selected from the group consisting of: Chinese Hamster Ovary (CHO) cells, Monkey Kidney (COS) cells, Human Embryonic Kidney (HEK-293) cells, and Human Cervical Cancer (HeLa) cells.

In some embodiments, the host cell is a mammalian host cell.

In yet another aspect, provided herein is a pharmaceutical composition comprising the antibody or antigen-binding fragment of the present invention, the nucleic acid molecule of the present invention, the expression vector of the present invention, or the host cell of the present invention, and a pharmaceutically acceptable carrier.

In yet another aspect, provided herein is the use of the antibody or antigen-binding fragment of the present invention, the nucleic acid molecule of the present invention, the expression vector of the present invention, the host cell of the present invention, or the pharmaceutical composition of the present invention in the manufacture of a medicament for treating a cell proliferative disease or delaying the progression of a cell proliferative disease.

In yet another aspect, provided herein is a method for treating a cell proliferative disease or delaying the progression of a cell proliferative disease, the method comprising administering to a patient in need thereof an effective amount of the antibody or antigen-binding fragment of the present invention, the nucleic acid molecule of the present invention, the expression vector of the present invention, the host cell of the present invention, or the pharmaceutical composition of the present invention.

In yet another aspect, provided herein is a method for treating cancer, the method comprising administering to a patient in need thereof an effective amount of the antibody or antigen-binding fragment of the present invention, the nucleic acid molecule of the present invention, the expression vector of the present invention, the host cell of the present invention, or the pharmaceutical composition of the present invention.

In some embodiments, the administration of the present invention is via oral, nasal, intravenous, subcutaneous, sublingual, or intramuscular route.

In some embodiments, the cell proliferative disease of the present invention is cancer.

In some embodiments, the cancer of the present invention is Ly6G6D-positive cancer.

In some embodiments, the Ly6G6D-positive cancer of the present invention is colorectal cancer, head and neck cancer, ovarian cancer, cervical cancer, melanoma, gastric cancer, esophageal cancer, small intestine cancer, large intestine cancer, or adenocarcinoma, etc.

In some embodiments, the adenocarcinoma of the present invention is colorectal adenocarcinoma, gastric adenocarcinoma, or pancreatic adenocarcinoma.

In yet another aspect, provided herein is an antibody-drug conjugate, the antibody-drug conjugate comprising the antibody or antigen-binding fragment of the present invention.

In some embodiments, the drug conjugate of the present invention is preferably a small molecule drug, wherein the antibody in the antibody-drug conjugate targets the small molecule drug to a therapeutic target via specific binding.

In yet another aspect, provided herein is a kit for detecting Ly6G6D, the kit of the present invention comprising the antibody or antigen-binding fragment of the present invention.

In some embodiments, the kit of the present invention further comprises containers, instructions for use, buffers, and the like. In the kit of the present invention, the antibody of the present invention may be immobilized on a detection plate.

### Definition of Term

In the present invention, unless otherwise specified, scientific and technical terms used herein shall have the meanings commonly understood by those skilled in the art. Furthermore, the operational steps for cell culture, molecular biology, biochemistry, nucleic acid chemistry, immunology, and the like used herein are conventional steps widely used in the respective fields. Meanwhile, to better understand the present invention, definitions and explanations of relevant terms are provided below.

When the terms "for example," "such as," "including," "comprising," or variants thereof are used herein, these terms shall not be considered limiting but shall be interpreted to mean "but not limited to" or "not limited to."

Unless otherwise indicated herein or clearly contradicted by context, the terms "a", "an", "the" and similar referents in the context of describing the present invention (especially in the context of the following claims) shall be construed to cover both the singular and the plural.

The terms "anti-Ly6G6D antibody" and "antibody that binds Ly6G6D" refer to an antibody that binds Ly6G6D with sufficient affinity such that the antibody is useful as a diagnostic and/or therapeutic agent targeting Ly6G6D.

The term "antibody" is used in its broadest sense and covers various antibody structures, including but not limited to monoclonal antibodies, polyclonal antibodies, multispecific antibodies (e.g., bispecific antibodies), and antibody fragments (e.g., dual Fab), provided they exhibit the desired antigen-binding activity.

The term "complementarity determining region" or "CDR" refers to amino acid residues in the antibody variable region responsible for antigen binding. Nanobodies contain three CDRs, designated CDR1, CDR2, and CDR3. The precise boundaries of these CDRs may be defined according to various numbering systems known in the art, for example, as defined in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given nanobody, those skilled in the art will readily identify the CDRs defined by each numbering system. Furthermore, the correspondence between different numbering systems is well known to those skilled in the art (see, e.g., Lefranc et al., Dev. Comparat. Immunol. 27:55-77, 2003).

The term "framework region" or "FR" residues refers to those amino acid residues in the antibody variable region other than the CDR residues as defined above. The FR of a variable domain typically consists of the following four FR domains: FR1, FR2, FR3, and FR4.

The term "specific binding" refers to a non-random binding reaction between two molecules, such as the reaction between an antibody and its target antigen. The strength or affinity of a specific binding interaction can be expressed by the equilibrium dissociation constant(KD) of said interaction. In the present invention, the term " KD" refers to the dissociation equilibrium constant of a specific antibody- antigen interaction, which is used to describe the binding affinity between the antibody and the antigen. The smaller the equilibrium dissociation constant, the tighter the antibody- antigen binding and the higher the affinity between the antibody and the antigen.

The term "affinity" refers to the strength of the sum of non-covalent interactions between a single binding site of a molecule (e.g., an antibody) and its binding partner (e.g., an antigen). Unless otherwise stated, as used herein, "binding affinity" refers to intrinsic binding affinity, which reflects the 1:1 interaction between members of a binding pair (e.g., an antibody and an antigen). The affinity of a molecule X for its partner Y can generally be expressed by the dissociation constant (KD). Affinity can be measured by conventional methods known in the art, including those described herein.

The terms "full-length antibody," "intact antibody," and "whole antibody" are used interchangeably herein to refer to an antibody having a structure substantially similar to that of a native antibody or having a heavy chain containing an Fc region as defined herein.

The term "native antibody" refers to naturally occurring immunoglobulin molecules with varying structures. For example, a native IgG antibody is a heterotetrameric glycoprotein of about 150,000 Daltons, composed of two identical light chains and two identical heavy chains linked by disulfide bonds. From the N-terminus to the C-terminus, each heavy chain has a variable region (VH), also referred to as the variable heavy chain domain or heavy chain variable domain, followed by three constant domains (CH1, CH2, and CH3). Similarly, from the N-terminus to the C-terminus, each light chain has a variable region (VL), also referred to as the variable light chain domain or light chain variable domain, followed by a constant light chain (CL) domain. Based on the amino acid sequence of their constant domains, the light chains of antibodies can be assigned to one of two types, referred to as kappa (κ) and lambda (λ).

The terms "antibody fragment" and "antigen-binding fragment" are interchangeable and refer to molecules, other than intact antibodies, that comprise a portion of an intact antibody and bind to the antigen to which the intact antibody binds. Examples of antibody fragments include, but are not limited to: dual Fab; Fv; Fab; Fab, Fab'-SH; F(ab')2; single-chain antibody molecules (e.g., scFv, ScFab); and multispecific antibodies formed from antibody fragments.

A Fab fragment is an antigen-binding fragment produced by papain digestion of an antibody and consists of an intact L chain and the variable region domain (VH) and first constant domain (CH1) of one H chain. Papain digestion of an antibody yields two identical Fab fragments. Pepsin treatment of an antibody yields a single large F(ab')2 fragment, which roughly corresponds to two Fab fragments linked by disulfide bonds, possessing bivalent antigen-binding activity and still capable of crosslinking antigens. A Fab' fragment differs from a Fab fragment in that the Fab' fragment has additional residues added to the carboxyl terminus of the CH1 domain, including one or more cysteines from the antibody hinge region. Fab'-SH is the designation herein for Fab' fragments wherein the cysteine residues of the constant domains bear free thiol groups. F(ab')2 antibody fragments were originally produced as pairs of Fab' fragments having hinge cysteines between them. Other chemical couplings of antibody fragments are also known.

"Fv" consists of a dimer of one heavy chain variable region domain and one light chain variable region domain in tight, non-covalent association. The folding of these two domains generates six hypervariable loops (three loops from the H chain and three from the L chain), which contribute amino acid residues for antigen binding and confer antigen-binding specificity to the antibody. However, even a single variable domain (or half of an Fv, containing only three CDRs specific for an antigen) retains the ability to recognize and bind the antigen, although its affinity is often lower than that of the intact binding site.

The term "Fc region" is used to define the C-terminal region of an immunoglobulin heavy chain, which includes native sequence Fc regions and variant Fc regions. Although the boundaries of the immunoglobulin heavy chain Fc region may vary, the human IgG heavy chain Fc region is typically defined as extending from the amino acid residue at position Cys226 or from Pro230 to the carboxyl terminus of the heavy chain. The C-terminal lysine of the Fc region (residue 447 according to the EU numbering system) may be removed, for example, during antibody production or purification, or through recombinant design of the nucleic acid encoding the antibody heavy chain. Accordingly, compositions of intact antibodies may include antibody populations from which all Lys447 residues have been removed, antibody populations from which Lys447 residues have not been removed, and antibody populations comprising a mixture of antibodies with and without Lys447 residues.

Light chains (LC) derived from any vertebrate can be assigned to one of two distinct types, designated κ and λ, based on the amino acid sequence of their constant domains. Immunoglobulins can be assigned to different classes or isotypes based on the amino acid sequence of their heavy chain constant domains (CH). There are five classes of immunoglobulins: IgA, IgD, IgE, IgG, and IgM, having heavy chains designated α, δ, γ, ε, and µ, respectively. The γ and α classes are further divided into subclasses based on relatively minor differences in CH sequence and function; for example, humans express the following subclasses: IgG1, IgG2, IgG3, IgG4, IgA1, and IgA2.

The term "chimeric" antibody refers to an antibody in which a portion of the heavy chain and/ or light chain is derived from a particular source or species, while the remainder of the heavy chain and/ or light chain is derived from a different source or species.

The term "human antibody" refers to an antibody having an amino acid sequence corresponding to that of an antibody produced by a human or human cell, or derived from an antibody utilizing a human antibody repertoire or other human antibody coding sequences from a non-human source. This definition of a human antibody specifically excludes humanized antibodies comprising non-human antigen-binding residues.

The term "humanized" antibody refers to a chimeric antibody comprising amino acid residues from non-human HVRs and amino acid residues from human FRs. In certain aspects, a humanized antibody will substantially comprise all of at least one, and typically two, variable domains, wherein all or substantially all of the HVRs (e.g., CDRs) correspond to HVRs of a non-human antibody, and all or substantially all of the FRs correspond to FRs of a human antibody. In certain aspects wherein all or substantially all of the FRs of a humanized antibody correspond to FRs of a human antibody, any FR of the humanized antibody may contain one or more amino acid residues from a non-human FR (e.g., one or more guide position residues of the FR). A humanized antibody optionally may comprise at least a portion of an antibody constant region derived from a human antibody. An antibody in "humanized form," for example, a non-human antibody, refers to an antibody that has undergone humanization.

The term "variable region" or "variable domain" refers to the domain of an antibody heavy or light chain that is involved in antibody-antigen binding. The variable domains of the heavy and light chains of natural antibodies (VH and VL, respectively) typically have similar structures, with each domain comprising four conserved framework regions (FRs) and three hypervariable regions (HVRs). (See, e.g., Kindt et al., Kuby Immunology, 6th ed., W.H. Freeman and Co., p. 91 (2007).) A single VH or VL domain may be sufficient to confer antigen-binding specificity. Furthermore, antibodies binding a particular antigen can be isolated using the VH or VL domain from an antibody binding that antigen, respectively, to screen libraries of complementary VL or VH domains. See, e.g., Portolano et al., J. Immunol. 150:880-887, 1993; Clarkson et al., Nature 352:624-628, 1991.

The term "hypervariable region" or "HVR" refers to each of the regions of an antibody variable domain that are hypervariable in sequence (complementarity determining regions or CDRs). Typically, an antibody comprises six CDRs; three in the VH (H-CDR1, H-CDR2, H-CDR3), and three in the VL (L-CDR1, L-CDR2, L-CDR3).

The term "single chain Fv," also abbreviated as "sFv" or "scFv," is an antibody fragment comprising VH and VL antibody domains linked into a single polypeptide chain. Preferably, the scFv polypeptide further comprises a polypeptide linker between the VH and VL domains, enabling the scFv to form the desired antigen-binding structure.

The term "monoclonal antibody" refers to an antibody obtained from a substantially homogeneous population of antibodies, i.e., the individual antibodies comprising the population are identical and/or bind the same epitope, except for possible variant antibodies (e.g., containing naturally occurring mutations or arising during the production of the monoclonal antibody preparation, which variants are typically present in minor amounts). In contrast to polyclonal antibody preparations, which typically include different antibodies directed against different determinants (epitopes), each monoclonal antibody in a monoclonal antibody preparation is directed against a single determinant on the antigen. Thus, the modifier "monoclonal" indicates the character of the antibody as being obtained from a substantially homogeneous population of antibodies, and is not to be construed as requiring production of the antibody by any particular method. For example, monoclonal antibodies for use according to the present invention can be prepared by a variety of techniques, including but not limited to hybridoma methods, recombinant DNA methods, phage display methods, and methods utilizing transgenic animals containing all or part of human immunoglobulin loci; such methods and other exemplary methods for preparing monoclonal antibodies are described herein.

The term "bispecific" antibody refers to an artificial antibody or antigen-binding fragment having specificity derived from two different monoclonal antibodies and capable of binding to two different epitopes. The two epitopes may be present on the same antigen, or they may be present on two different antigens.

The term "multispecific antibody" is used in its broadest sense and specifically encompasses antibodies having multispecificity. In one aspect, a multispecific antibody binds to two different targets (e.g., a bispecific antibody). Such multispecific antibodies include, but are not limited to, antibodies comprising a heavy chain variable domain (VH) and a light chain variable domain (VL), wherein the VH/VL unit has multispecificity; antibodies having two or more VL and VH domains, wherein each VH/VL unit binds to a different epitope; antibodies having two or more single variable domains, wherein each single variable domain binds to a different epitope; full-length antibodies; and antibody fragments such as Fab, Fv, dsFv, scFv, diabodies, bispecific diabodies, and tribodies, which may be covalently or non-covalently linked.

The term "tumor" refers to all neoplastic cell growth and proliferation, whether malignant or benign, as well as all pre-cancerous and cancerous cells and tissues. The terms "cancer," "cancerous," "cell proliferative disorder," "cell proliferative disease," "proliferative disorder," and "tumor" are not mutually exclusive herein.

The terms "cancer" and "cancerous" refer to or describe a physiological condition in mammals that is typically characterized by uncontrolled cell growth/proliferation. Aspects of cancer include solid tumor cancers and non-solid tumor cancers. Solid tumor cancers include, but are not limited to, colorectal cancer, melanoma, breast cancer, lung cancer, head and neck cancer, bladder cancer, kidney cancer, ovarian cancer, pancreatic cancer, or prostate cancer, or metastatic forms thereof. The cancer may be LY6G6D-positive cancer.

In some aspects, the cancer is colorectal cancer. As used herein, the terms "colorectal cancer," "CRC," "colon cancer," or "bowel cancer" refer to cancers arising from the large intestine, such as the colon or rectum.

"Effector function" refers to those biological activities attributable to the Fc region of an antibody that vary with the antibody isotype. Examples of antibody effector functions include: C1q binding and complement-dependent cytotoxicity(CDC); Fc receptor binding; antibody-dependent cell-mediated cytotoxicity(ADCC); phagocytosis; downregulation of cell surface receptors ( e.g., B cell receptors); and B cell activation.

" Complement-dependent cytotoxicity" or" CDC" refers to the lysis of target cells in the presence of complement. Activation of the classical complement pathway is initiated by the binding of the first component of the complement system(C1q) to an antibody (of an appropriate subclass) bound to its homologous antigen.

"Antibody-dependent cell-mediated cytotoxicity" or "ADCC" refers to a form of cytotoxicity in which secreted Ig binds to Fc receptors (FcRs) present on certain cytotoxic cells (e.g., natural killer (NK) cells, neutrophils, and macrophages), enabling these cytotoxic effector cells to specifically bind to antigen-bearing target cells, subsequently killing the target cells with cytotoxic agents. Antibodies "arm" cytotoxic cells and are required for such killing. The primary cells mediating ADCC, NK cells, express only FcγRIII, whereas monocytes express FcγRI, FcγRII, and FcγRIII.

The term "antibody-drug conjugate (ADC)" refers to the antibody conjugated, and preferably chemically conjugated, to the effector molecule. Wherein, the effector molecule is preferably a therapeutically active drug, such as one or more of a toxin, a chemotherapeutic agent, a small molecule drug, or a radionuclide.

As used herein, "delaying progression" of a condition or disease means slowing, hindering, retarding, delaying, stabilizing, and/or postponing the development of the disease or condition (e.g., a cell proliferative disorder, e.g., cancer). Such delay may vary in duration depending on the medical history and/or the individual to be treated. It will be apparent to those skilled in the art that sufficient or significant delay may effectively encompass prevention, as the individual does not develop the disease. For example, the development of advanced cancer, such as metastasis, may be delayed.

The term "treatment" (and its grammatical variations, such as "treat" or "treating") refers to a clinical intervention intended to alter the natural course of the individual being treated, and may be for the purpose of prevention or during the course of clinical pathology. Desired effects of treatment include, but are not limited to, preventing the occurrence or recurrence of the disease, alleviating symptoms, diminishing any direct or indirect pathological consequences of the disease, preventing metastasis, decreasing the rate of disease progression, ameliorating or palliating the disease state, and remitting or improving prognosis. In some aspects, the antibodies of the present invention (e.g., the anti-LY6G6D antibodies of the present invention) are used to delay the development of the disease or slow the progression of the disease.

The "effective amount" of a compound (e.g., an anti-LY6G6D antibody of the present invention) or a composition thereof (e.g., a pharmaceutical composition) is at least the minimum amount required to achieve a desired therapeutic or prophylactic outcome, such as a measurable improvement or prevention of a specific disorder (e.g., a cell proliferative disorder, e.g., cancer). The effective amount herein may vary depending on factors such as the patient's disease state, age, sex, and weight, as well as the ability of the antibody to elicit the intended response in the individual. An effective amount is also an amount in which the therapeutically beneficial effects outweigh any toxic or detrimental effects of the treatment. For prophylactic uses, beneficial or intended outcomes include, for example, elimination or reduction of risk, alleviation of severity, or delay of disease onset, encompassing biochemical, histological, and/or behavioral symptoms of the disease, its complications, and intermediate pathological phenotypes arising during the course of disease development. For therapeutic uses, beneficial or intended outcomes include clinical results such as reduction of one or more symptoms caused by the disease, improvement in the quality of life of affected individuals, reduction in the dosage of other drugs required to treat the disease, and enhancement of the effects of other drugs (such as through targeting, delaying disease progression, and/or prolonging survival). In the case of cancer or tumors, an effective amount of the drug may reduce the number of cancer cells; decrease tumor size; inhibit (i.e., slow to some extent or is expected to stop) infiltration of cancer cells into surrounding organs; inhibit (i.e., slow to some extent and is expected to stop) tumor metastasis; inhibit tumor growth to some extent; and/or alleviate one or more symptoms associated with the condition to some extent. The effective amount may be administered once or multiple times. For the purposes of the present invention, an effective amount of a drug, compound, or pharmaceutical composition is an amount sufficient to directly or indirectly achieve prophylaxis or treatment. As understood in a clinical context, an effective amount of a drug, compound, or pharmaceutical composition may or may not be achieved in combination with another drug, compound, or pharmaceutical composition. Therefore, an "effective amount" may be considered in the context of administering one or more therapeutic agents, and an effective amount of a single agent may be considered if the intended outcome can be obtained or achieved in combination with one or more other agents.

An "isolated" protein or peptide is a protein or peptide that has been separated from components of its natural environment. In some aspects, the protein or peptide is purified to greater than 95% or 99% purity, as determined by, for example, electrophoresis (e.g., SDS-PAGE, isoelectric focusing (IEF), capillary electrophoresis) or chromatography (e.g., ion exchange or reverse-phase HPLC). An "isolated" nucleic acid refers to a nucleic acid molecule that has been separated from components of its natural environment. Isolated nucleic acids include nucleic acid molecules that are contained within a cell that normally contains said nucleic acid molecule, but wherein said nucleic acid molecule is present extrachromosomally or at a chromosomal location different from its native chromosomal location.

The term "expression vector" refers to a nucleic acid vehicle into which a polynucleotide can be inserted. When a vector enables the expression of a protein encoded by an inserted polynucleotide, the vector is termed an expression vector. Vectors can be introduced into host cells via transformation, transduction, or transfection, enabling the genetic material elements they carry to be expressed in the host cells. Vectors are well known to those skilled in the art and include, but are not limited to: plasmids; phagemids; cosmids; artificial chromosomes, such as yeast artificial chromosomes (YAC), bacterial artificial chromosomes (BAC), or P1-derived artificial chromosomes (PAC); bacteriophages such as lambda phage or M13 phage; and animal viruses. Animal viruses that can be used as vectors include, but are not limited to, retroviruses (including lentiviruses), adenoviruses, adeno-associated viruses, herpesviruses (such as herpes simplex virus), poxviruses, baculoviruses, papillomaviruses, and papovaviruses (such as SV40). A vector may contain multiple elements controlling expression, including but not limited to promoter sequences, transcription initiation sequences, enhancer sequences, selection elements, and reporter genes. Additionally, vectors may contain an origin of replication.

The term "nucleic acid molecule" is intended to include DNA molecules and RNA molecules. Nucleic acid molecules may be single-stranded or double-stranded and may be cDNA.

The term "host cell" refers to a cell that can be used to introduce a vector, including but not limited to prokaryotic cells such as Escherichia coli or Bacillus subtilis; fungal cells such as yeast cells or Aspergillus species; insect cells such as Drosophila S2 cells or Sf9 cells; or animal cells such as fibroblasts, CHO cells, COS cells, NSO cells, HeLa cells, BHK cells, HEK 293 cells, or human cells. Host cells may include single cells or cell populations.

The term "identity" or "sequence identity" refers to the degree of match between sequences of two polypeptides or two nucleic acids. When a specific position in both sequences being compared is occupied by the same base or amino acid monomeric subunit (for example, when a specific position in each of two DNA molecules is occupied by adenine, or a specific position in each of two polypeptides is occupied by lysine), then the molecules are identical at that position. The "percent identity" between two sequences is a function of the number of matching positions shared by the two sequences divided by the number of positions compared, multiplied by 100. For example, if 6 out of 10 positions match in two sequences, then these two sequences have 60% identity. Typically, comparisons are made when two sequences are aligned to generate maximum identity. Such alignment can be achieved using, for example, the method of Needleman et al. (1970) J. Mol. Biol. 48:443-453, which can be conveniently performed via computer programs such as the Align program (DNAstar, Inc.). The percent identity between two amino acid sequences can also be determined using the algorithm of E. Meyers and W. Miller (Comput. Appl. Biosci., 4:11-17 (1988)) integrated into the ALIGN program (version 2.0), employing a PAM120 weight residue table, a gap length penalty of 12, and a gap penalty of 4. Furthermore, the percent identity between two amino acid sequences can be determined using the Needleman and Wunsch (J. Mol. Biol. 48:444-453 (1970)) algorithm in the GAP program integrated into the GCG software package (available at www.gcg.com), employing a BLOSSUM 62 matrix or a PAM250 matrix, along with gap weights of 16, 14, 12, 10, 8, 6, or 4, and length weights of 1, 2, 3, 4, 5, or 6.

The term "pharmaceutically acceptable carrier" refers to a carrier that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes but is not limited to: pH adjusters, surfactants, adjuvants, ionic strength enhancers, diluents, agents for maintaining osmotic pressure, agents for delaying absorption, and preservatives. For example, pH adjusters include but are not limited to phosphate buffers. Surfactants include but are not limited to cationic, anionic, or nonionic surfactants, such as Tween-80. Ionic strength enhancers include but are not limited to sodium chloride. Preservatives include but are not limited to various antibacterial and antifungal agents, such as parabens, chlorobutanol, phenol, sorbic acid, etc. Agents for maintaining osmotic pressure include but are not limited to sugars, NaCl, and analogs thereof. Agents for delaying absorption include but are not limited to monostearates and gelatin. Diluents include but are not limited to water, aqueous buffers (such as buffered saline), alcohols, and polyols (such as glycerin), etc. Preservatives include but are not limited to various antibacterial and antifungal agents, such as thimerosal, 2-phenoxyethanol, parabens, chlorobutanol, phenol, sorbic acid, etc. Stabilizers have the meaning commonly understood by those skilled in the art and are capable of stabilizing the desired activity of the active ingredient in the drug, including but not limited to monosodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein), or degradation products thereof (such as lactalbumin hydrolysate), etc. In certain exemplary embodiments, the pharmaceutically acceptable carrier includes sterile injectable liquids (such as aqueous or nonaqueous suspensions or solutions). In some exemplary embodiments, such sterile injectable liquids are selected from Water for Injection (WFI), Bacteriostatic Water for Injection (BWFI), sodium chloride solution (e.g., 0.9% (w/v) NaCl), glucose solution (e.g., 5% glucose), solutions containing surfactants (e.g., 0.01% polysorbate 20), pH buffer solutions (e.g., phosphate buffer solution), Ringer's solution, and any combination thereof.

The term "subject" refers to a mammal, such as a primate mammal, for example, a human. In some embodiments, the subject (e.g., a human) suffers from a disease related to Ly6G6D.

The term "administration" refers to a method of delivering a certain dose of a compound (e.g., the anti- LY6G6D antibody of the present invention) to a subject. In some aspects, the compositions used in the methods described herein are administered intravenously. The compositions used in the methods described herein may be administered, for example, intramuscularly, intravenously, intradermally, transdermally, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subcutaneously, subconjunctivally, intravesicularly, mucosally, pericardially, intraumbilically, intraocularly, orally, externally, locally, by inhalation, by injection, by infusion, by continuous infusion, by local perfusion directly lavaging target cells, by catheter, by lavage, in the form of an emulsion, or in the form of a lipid composition. The method of administration may vary depending on various factors (e.g., the compound or composition to be administered and the severity of the condition, disease, or disorder to be treated).

The embodiments of the present invention will be described in detail below with reference to the accompanying drawings and examples. However, those skilled in the art will understand that the following drawings and examples are for illustrative purposes only and do not limit the scope of the present invention. Various objects and advantageous aspects of the present invention will become apparent to those skilled in the art from the following detailed description of the accompanying drawings and preferred embodiments.

### Description of the drawings

Figure 1 is a graph showing the binding results of candidate antibodies to the hLY6G6D antigen confirmed by ELISA;
Figure 2 is a graph showing the binding results of candidate antibodies to the cynoLY6G6D antigen confirmed by ELISA;
Figure3 is a graph showing the binding results of candidate antibodies to HEK293T cells highly expressing hLY6G6D detected by flow cytometry;
Figure4 is a graph showing the binding results of candidate antibodies to HEK293T cells highly expressing cynoLY6G6D detected by flow cytometry;
Figure 5 is a graph showing the results of evaluating the ADCC activity of candidate molecules using the Jurkat-NFAT-Luc2-CD16 cell model.

### EXAMPLES

The scheme of the present invention will be explained below with reference to embodiments. Those skilled in the art will appreciate that the following embodiments are only used to illustrate the present invention and should not be construed as limiting the scope of the present invention. If the specific technology or conditions are not indicated in the examples, the technology or conditions described in the literature in the art or the product descriptions are performed. If the reagents or instruments used are not specified by the manufacturers, they are all conventional products that are commercially available.

Unless otherwise specified, the molecular biology experimental methods and immunoassays used in the present invention are basically performed according to the methods described in J. Sambrook et al., Molecular Cloning: A Laboratory Manual, 2nd Edition, Cold Spring Harbor Laboratory Press, 1989, and F. M. Ausubel et al., Current Protocols in Molecular Biology, 3rd Edition, John Wiley & Sons, Inc., 1995. Those skilled in the art will appreciate that the examples describe the present invention by way of illustration and are not intended to limit the scope of the claimed invention.

### Example 1: Screening of LY6G6D monoclonal antibodies using phage display technology and flow cytometry

High-affinity monoclonal phages binding to LY6G6D at the molecular level were screened by panning a natural fully human phage display library combined with the ELISA method. The genes encoding the extracellular domains of HumanLY6G6D and cynoLY6G6D proteins were designed using the Uniprot and NCBI databases, constructed into a mammalian eukaryotic expression system, and expressed to obtain the extracellular domains of HumanLY6G6D and cynoLY6G6D proteins for screening the natural human antibody library. The heavy chain variable region and light chain variable region sequences of antibodies were amplified from human PBMCs. The heavy and light chain gene fragments were connected via a L inker (specifically, the L inker has the amino acid sequence shown in SEQ ID NO: 112) to establish a natural human antibody phage library. The library was panned using HumanLY6G6D protein. After four rounds of panning, monoclonal phages capable of binding to HumanLY6G6D were screened using the ELISA method. The binding results of 16 candidate phages to HumanLY6G6D are shown in Table 1 below. The results in Table 1 indicate that all 16 candidate phages of the present invention exhibit good binding to HumanLY6G6D.

**Table 1**

| **No.** | 2E7 | 2G10 | 5C12 | 6C1 | 7B1 | 12A1 | 13A10 | 14A2 |
|---|---|---|---|---|---|---|---|---|
| **OD450** | 1.248 | 2.269 | 2.237 | 1.244 | 1.257 | 1.879 | 0.963 | 1.452 |
| **No.** | 14G2 | 14B5 | 16H6 | 17A11 | 18A11 | 19A4 | 20E2 | 22A6 |
| **OD450** | 1.254 | 0.838 | 1.16 | 1.19 | 1.551 | 1.425 | 0.989 | 1.412 |

### Example 2: Expression and purification of antibodies

The 16 candidate phages obtained in Example 1 were sequenced to obtain the variable region sequences of the light and heavy chains encoding the antibody candidates. Single-chain antibody gene coding sequences (VH-Linker1-VL-Linker2-Fc, wherein VH has the amino acid sequence shown in any one of SEQ ID NO: 77-92, VL has the amino acid sequence shown in any one of SEQ ID NO: 93-108, Linker1 and Linker2 have the amino acid sequence shown in SEQ ID NO: 112, and Fc has the amino acid sequence shown in SEQ ID NO: 114) were synthesized using whole gene synthesis methods. A series of antibody expression vectors were constructed using molecular cloning methods and expressed recombinantly in the 293F expression system. After resuscitation and culture of 293F host cells in OPMI medium, cells were collected at a cell density of approximately 3*10⁶ cell/mL and transfected using PEI reagent. On the second day of culture, feed and glucose were added to achieve a culture concentration of 8 g/L. On the sixth day of culture, the cell culture supernatant was collected.

A series of candidate antibodies were detected at the translation level. The collected cell culture supernatant was purified using a Protein A chromatography column, and absorption peaks were collected for mass spectrometry analysis. Mass spectrometry analysis showed that the molecular weight of the candidate antibody scFv-Fc series was around 100 kDa, consistent with the theoretical molecular weight, indicating a dimeric form. Meanwhile, the collected samples were analyzed by 10% SDS-PAGE electrophoresis after reduction and non-reduction. The reduced SDS-PAGE electropherogram of the candidate antibody scFv-Fc showed bands around 50 kDa, while the nonreduced SDS-PAGE electropherogram showed a single band around 100 kDa; the band sizes in the electropherograms were consistent with theory. The purified samples were dialyzed overnight at 4 °C against 0.02 M PBS buffer at pH 7.4.

### Example 3: Detection of Binding Characteristics Between Antibodies and Antigen

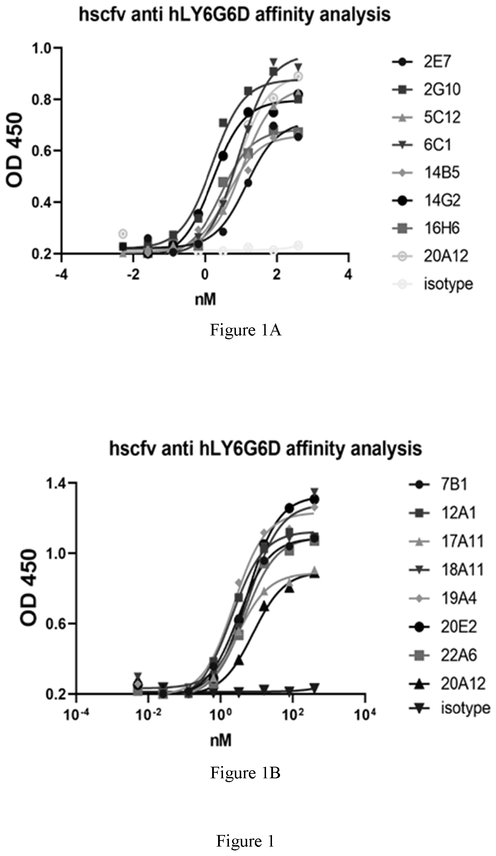

The binding of candidate antibodies obtained in Example 2 to the hLY6G6D antigen was confirmed by ELISA. ELISA plates were coated with 100 ng/well of hLY6G6D and incubated in an electric thermostatic incubator at 37°C for 2 h. Non-specific binding sites were then blocked overnight with 5% skim milk powder at 4°C. The plates were incubated with mAbs at different concentrations for 1 h, washed three times with 0.1% PBST, and HRP-mouse anti-human IgG1 Fc was added to detect the bound mAbs. After washing five times with 0.1% PBST, TMB substrate (Yunqiao Bio) was added. The reaction was stopped after color development, and readings were taken at 450 nm. The results are shown in Figure 1 and Tables 2-3, wherein Figure 1A corresponds to Table 2, and Figure 1B corresponds to Table 3. The affinity of the candidate antibodies was comparable to that of the positive antibody 20A12, and among them, 17A11, 19A4, and 20E2 exhibited superior binding activity to the hLY6G6D antigen compared to the positive antibody 20A12. The positive control antibody 20A12 is derived from patent CN202080086360.1; the heavy chain of antibody 20A12 comprises the amino acid sequence shown in SEQ ID NO:115, and the light chain of antibody 20A12 comprises the amino acid sequence shown in SEQ ID NO:116;

EVQLLESGGGLVQPGGSLRLSCAASGFDFVNNAMIWVRQAPGKGLEWVSALSFADNT
AYYATWASGRFTISRDSSKTTVYLQMNSLRAEDTAVYYCMRGDLWGPGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLV

KGFYPSDIAVEWESNGQPENNYKTTPPVLDSDGSFFLYSKLTVDKSRWQQGNVFSCSVMHE
ALHNHYTQKSLSLSPGK (SEQ ID NO: 115),

DIQMTQSPSTLSASVGDRVTITCQASESITRYLNWYQQKPGKAPKLLIYDASKLPSGVP
SRFSGSGSGTEFTLTISSLQPDDFATYYCQSTSFRGRSYQNTFGGGTKVEIKRTVAAPSVFIFP
PSDEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO: 116).

**Table 2**

| No. | 2E7 | 2G10 | 5C12 | 6C1 | 14B5 | 14G2 | 16H6 | 20A12 |
|---|---|---|---|---|---|---|---|---|
| EC50 (nM) | 14.27 | 1.605 | 10.78 | 8.882 | 4.383 | 1.667 | 2.873 | 7.89 |

**Table 3**

| No. | 7B1 | 12A1 | 17A11 | 18A11 | 19A4 | 20E2 | 22A6 | 20A12 |
|---|---|---|---|---|---|---|---|---|
| EC50 (nM) | 3.086 | 2.094 | 2.885 | 6.281 | 2.356 | 5.399 | 4.601 | 7.89 |

### Example 4: Detection of Binding Characteristics Between Antibodies and Antigen

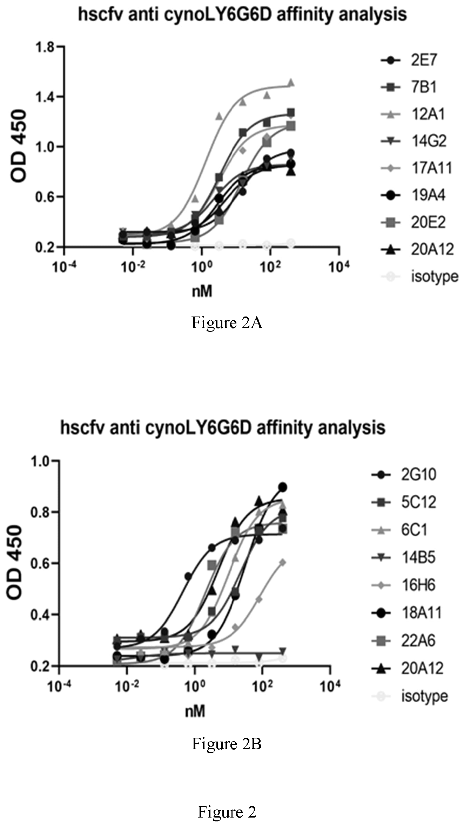

Cross-reactivity with cynomolgus monkey: The binding of expressed candidate antibodies (obtained in Example 2) to cynoLY6G6D antigen was confirmed by ELISA. Information on the positive control antibody 20A12 is as shown in Example 3. ELISA plates were coated with 100 ng/well of cynoLY6G6D and incubated in an electric thermostatic incubator at 37°C for 2 h. Non-specific binding sites were then blocked overnight with 5% skim milk powder at 4°C. The plates were incubated with mAbs at different concentrations for 1 h, washed three times with 0.1% PBST, and HRP-mouse anti-human IgG1 Fc was added to detect the bound mAbs. After washing five times with 0.1% PBST, TMB substrate (Yunqiao Bio) was added. The reaction was stopped after color development, and readings were taken at 450 nm. The results are shown in Figure 2 and Tables 4-5; Figure 2A corresponds to Table 4, and Figure 2B corresponds to Table 5. Candidate antibody 14B5 showed no binding to cynoLY6G6D, while the remaining candidate antibodies and the positive antibody all exhibited binding to cynoLY6G6D.

**Table 4**

| No. | 2E7 | 7B1 | 12A1 | 14G2 | 17A11 | 19A4 | 20E2 | 20A12 |
|---|---|---|---|---|---|---|---|---|
| EC50 (nM) | 13.66 | 3.388 | 1.332 | 2.213 | 3.206 | 2.86 | 14.6 | 4.975 |

**Table 5**

| No. | 2G10 | 5C12 | 6C1 | 14B5 | 16H6 | 18A11 | 22A6 | 20A12 |
|---|---|---|---|---|---|---|---|---|
| EC50 (nM) | 0.4541 | 22.53 | 10.23 | 0.0204 | 86.34 | 31.53 | 2.047 | 4.975 |

### Example 5: Detection of Binding Characteristics Between Antibodies and Antigen

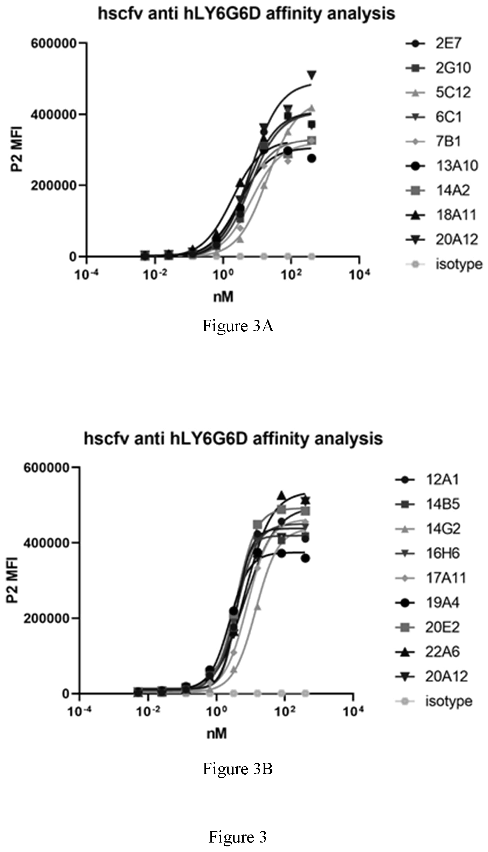

Flow cytometry was used to detect the binding of candidate antibodies (obtained in Example 2) to HEK293T cells highly expressing hLY6G6D. Information on the positive control antibody 20A12 is provided in Example 3. 2*10⁵ cells were resuspended in a 96-well plate and incubated with different concentrations of mAb for 1 h. The cells were then washed twice with PBS. PE-conjugated Goat anti-human IgG Fc (Jackson ImmunoResearch), diluted 1:500, was added and incubated at 4°C for 30 min to detect the bound candidate antibodies. Subsequently, the cells were washed twice with PBS and analyzed on a FACS instrument (Beckman). The results are shown in Figure 3 and Tables 6-7. Figure 3A corresponds to Table 6, and Figure 3B corresponds to Table 7. The affinity of the candidate antibodies was comparable to that of the positive control antibody 20A12, among which 19A4 and 20E2 exhibited superior binding activity to hLY6G6D-positive HEK293T cells compared to the positive control antibody 20A12.

**Table 6**

| No. | 2E7 | 2G10 | 5C12 | 6C1 | 7B1 | 13A10 | 14A2 | 18A11 | 20A12 |
|---|---|---|---|---|---|---|---|---|---|
| EC50 (nM) | 5.195 | 6.508 | 19.85 | 6.517 | 6.795 | 3.007 | 3.909 | 1.885 | 6.711 |

**Table 7**

| No. | 12A1 | 14B5 | 14G2 | 16H6 | 17A11 | 19A4 | 20E2 | 22A6 | 20A12 |
|---|---|---|---|---|---|---|---|---|---|
| EC50 (nM) | 3.937 | 3.789 | 14.09 | 3.957 | 7.907 | 2.463 | 3.907 | 7.022 | 6.893 |

### Example 6: Detection of Binding Characteristics Between Antibodies and Antigen

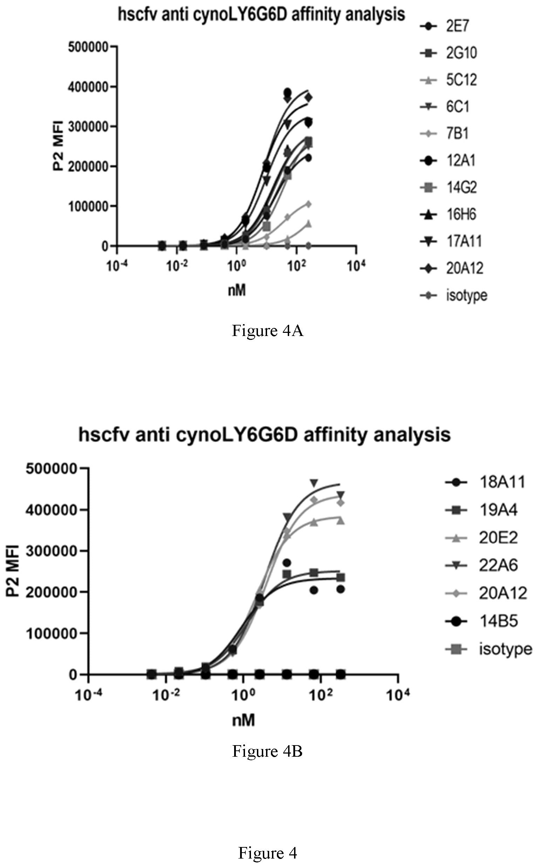

Cross-reactivity with cynomolgus monkey: Flow cytometry was used to detect the binding of candidate antibodies (obtained in Example 2) to HEK293T cells highly expressing cynoLY6G6D. Information on the positive control antibody 20A12 is provided in Example 3. 2*10⁵ cells were resuspended in a 96-well plate and incubated with different concentrations of mAb for 1 h. The cells were then washed twice with PBS. PE-conjugated Goat anti-human IgG Fc (Jackson ImmunoResearch), diluted 1:500, was added and incubated at 4°C for 30 min to detect bound antibodies. Subsequently, the cells were washed twice with PBS and analyzed on a FACS instrument (Beckman). The results are shown in Figure 4. Candidate antibody 14B5 did not bind to cynoLY6G6D, whereas the remaining candidate antibodies and the positive antibody all exhibited binding to cynoLY6G6D.

### Example 7: Evaluation of ADCC Activity of Candidate Molecules Using the Jurkat-NFAT-Luc2-CD16 Cell Model

A Jurkat-NFAT-Luc2-CD16 reporter gene cell model was constructed using Jurkat cells stably transfected with the FcγRIIIa receptor and NFAT (nuclear factor of activated T cells) to evaluate the ADCC activity of candidate molecules.

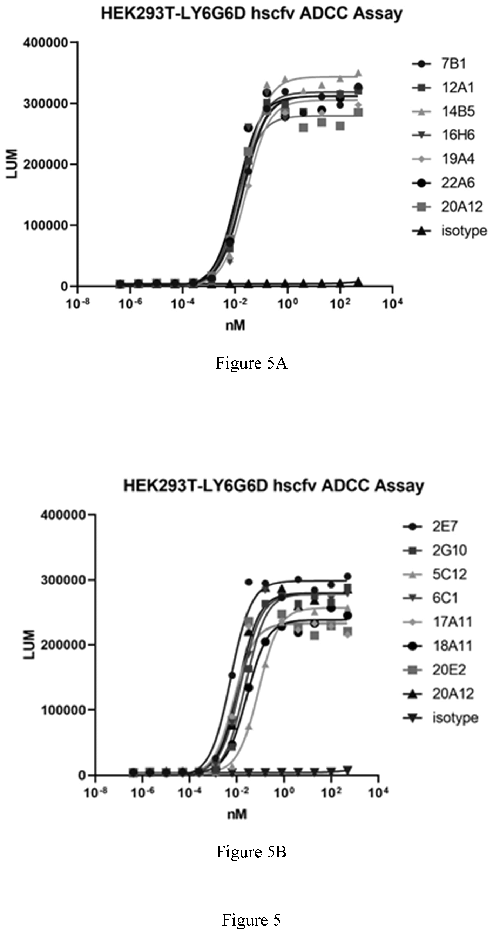

hLY6G6D-HEK293T cells constructed herein were used as target cells, and Jurkat-NFAT-Luc2-CD16 cells were used as effector cells. Effector cells and target cells were seeded in a 384-well plate at 5,000 cells per well each. Information on the positive control antibody 20A12 is provided in Example 3. Dilutions of antibodies at different concentrations (obtained in Example 2) were added to the 384-well plate. Effector cells and target cells were co-cultured in a 37 °C, 5% CO₂ incubator for 14 h. The antibodies specifically bound to hLY6G6D on the surface of target cells, and the Fc region of the antibodies bound to FcγRIIIa receptors on the surface of effector cells, thereby forming target cell-antibody-effector cell complexes. This activated the NFAT-Luc signaling pathway within the effector cells, leading to luciferase production. Upon addition of the luciferase detection substrate and a 3-min reaction, the luminescence value of each well was read using a multifunctional microplate reader. The results are shown in Figure 5 and Tables 8-9; Figure 5A corresponds to Table 8, and Figure 5B corresponds to Table 9. The ADCC activity of the candidate antibodies was comparable to that of the positive antibody 20A12, and the ADCC activity of 17A11 and 20E2 was superior to that of the positive antibody.

**Table 8**

| No. | 7B1 | 12A1 | 14B5 | 16H6 | 19A4 | 22A6 | 20A12 |
|---|---|---|---|---|---|---|---|
| EC50 (nM) | 0.0208 | 0.01893 | 0.02252 | 0.01525 | 0.02603 | 0.01231 | 0.01213 |

**Table 9**

| No. | 2E7 | 2G10 | 5C12 | 6C1 | 17A11 | 18A11 | 20E2 | 20A12 |
|---|---|---|---|---|---|---|---|---|
| EC50 (nM) | 0.005627 | 0.02368 | 0.08249 | 0.01506 | 0.007523 | 0.0265 | 0.007653 | 0.01213 |

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the above terms throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although specific embodiments of the present invention have been described in detail, those skilled in the art will understand that various modifications and changes to the details can be made based on all the teachings disclosed herein, and such changes fall within the scope of protection of the present invention. The full scope of the present invention is defined by the appended claims and any equivalents thereof. The full scope of the present invention is defined by the appended claims and any equivalents thereof.

## Claims

1. An anti-Ly6G6D antibody or antigen-binding fragment, **characterized in that** the antibody or antigen-binding fragment binds to Ly6G6D, and the antibody comprises a heavy chain and a light chain, wherein, the heavy chain comprises a heavy chain variable region, and the heavy chain variable region comprises complementarity determining regions: H-CDR1, H-CDR2 and H-CDR3; wherein,
(1) H-CDR1 has an amino acid sequence shown in SEQ ID NO: 1, SEQ ID NO: 7, SEQ ID NO: 13, SEQ ID NO: 18, SEQ ID NO: 22, SEQ ID NO: 28, SEQ ID NO: 34, SEQ ID NO: 42, SEQ ID NO: 46, SEQ ID NO: 52, SEQ ID NO: 56, SEQ ID NO: 61 or SEQ ID NO: 71; and
(2) H-CDR2 has an amino acid sequence shown in SEQ ID NO: 2, SEQ ID NO: 8, SEQ ID NO: 14, SEQ ID NO: 19, SEQ ID NO: 23, SEQ ID NO: 29, SEQ ID NO: 35, SEQ ID NO: 43, SEQ ID NO: 47, SEQ ID NO: 53, SEQ ID NO: 57 or SEQ ID NO: 72; and
(3) H-CDR3 has an amino acid sequence shown in SEQ ID NO: 3, SEQ ID NO: 9, SEQ ID NO: 15, SEQ ID NO: 20, SEQ ID NO: 24, SEQ ID NO: 30, SEQ ID NO: 36, SEQ ID NO: 40, SEQ ID NO: 44, SEQ ID NO: 48, SEQ ID NO: 54, SEQ ID NO: 58, SEQ ID NO: 62, SEQ ID NO: 66 or SEQ ID NO: 73; and
wherein, the light chain comprises a light chain variable region, the light chain variable region comprises complementarity determining regions: L-CDR1, L-CDR2 and L-CDR3; wherein,
(1) L-CDR1 has an amino acid sequence shown in SEQ ID NO: 4, SEQ ID NO: 10, SEQ ID NO: 25, SEQ ID NO: 31, SEQ ID NO: 37, SEQ ID NO: 49, SEQ ID NO: 59, SEQ ID NO: 63, SEQ ID NO: 68 or SEQ ID NO: 74; and
(2) L-CDR2 has an amino acid sequence shown in SEQ ID NO: 5, SEQ ID NO: 11, SEQ ID NO: 16, SEQ ID NO: 26, SEQ ID NO: 32, SEQ ID NO: 38, SEQ ID NO: 50, SEQ ID NO: 60, SEQ ID NO: 64, SEQ ID NO: 67, SEQ ID NO: 69 or SEQ ID NO: 75; and
(3) L-CDR3 has an amino acid sequence shown in SEQ ID NO: 6, SEQ ID NO: 12, SEQ ID NO: 17, SEQ ID NO: 21, SEQ ID NO: 27, SEQ ID NO: 33, SEQ ID NO: 39, SEQ ID NO: 41, SEQ ID NO: 45, SEQ ID NO: 51, SEQ ID NO: 55, SEQ ID NO: 65, SEQ ID NO: 70 or SEQ ID NO: 76.

2. The antibody or antigen-binding fragment according to claim 1, **characterized in that** the heavy chain variable region comprises complementarity determining regions: H-CDR1, H-CDR2 and H-CDR3; wherein,
(1) H-CDR1 has the amino acid sequence shown in SEQ ID NO:1, H-CDR2 has the amino acid sequence shown in SEQ ID NO:2 and H-CDR3 has the amino acid sequence shown in SEQ ID NO:3; or
(2) H-CDR1 has the amino acid sequence shown in SEQ ID NO:7, H-CDR2 has the amino acid sequence shown in SEQ ID NO:8 and H-CDR3 has the amino acid sequence shown in SEQ ID NO:9; or
(3) H-CDR1 has the amino acid sequence shown in SEQ ID NO:13, H-CDR2 has the amino acid sequence shown in SEQ ID NO:14 and H-CDR3 has the amino acid sequence shown in SEQ ID NO:15; or
(4) H-CDR1 has the amino acid sequence shown in SEQ ID NO:18, H-CDR2 has the amino acid sequence shown in SEQ ID NO:19 and H-CDR3 has the amino acid sequence shown in SEQ ID NO:20; or
(5) H-CDR1 has the amino acid sequence shown in SEQ ID NO:22, H-CDR2 has the amino acid sequence shown in SEQ ID NO:23 and H-CDR3 has the amino acid sequence shown in SEQ ID NO:24; or
(6) H-CDR1 has the amino acid sequence shown in SEQ ID NO:28, H-CDR2 has the amino acid sequence shown in SEQ ID NO:29 and H-CDR3 has the amino acid sequence shown in SEQ ID NO:30; or
(7) H-CDR1 has the amino acid sequence shown in SEQ ID NO:34, H-CDR2 has the amino acid sequence shown in SEQ ID NO:35 and H-CDR3 has the amino acid sequence shown in SEQ ID NO:36; or
(8) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:40; or
(9) H-CDR1 has the amino acid sequence shown in SEQ ID NO:42, H-CDR2 has the amino acid sequence shown in SEQ ID NO:43 and H-CDR3 has the amino acid sequence shown in SEQ ID NO:44; or
(10) H-CDR1 has the amino acid sequence shown in SEQ ID NO:46, H-CDR2 has the amino acid sequence shown in SEQ ID NO:47 and H-CDR3 has the amino acid sequence shown in SEQ ID NO:48; or
(11) H-CDR1 has the amino acid sequence shown in SEQ ID NO:52, H-CDR2 has the amino acid sequence shown in SEQ ID NO:53 and H-CDR3 has the amino acid sequence shown in SEQ ID NO:54; or
(12) H-CDR1 has the amino acid sequence shown in SEQ ID NO:56, H-CDR2 has the amino acid sequence shown in SEQ ID NO:57 and H-CDR3 has the amino acid sequence shown in SEQ ID NO:58; or
(13) H-CDR1 has the amino acid sequence shown in SEQ ID NO:61, H-CDR2 has the amino acid sequence shown in SEQ ID NO:29 and H-CDR3 has the amino acid sequence shown in SEQ ID NO:62; or
(14) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:66; or
(15) H-CDR1 has the amino acid sequence shown in SEQ ID NO:18, H-CDR2 has the amino acid sequence shown in SEQ ID NO:29 and H-CDR3 has the amino acid sequence shown in SEQ ID NO:62; or
(16) H-CDR1 has the amino acid sequence shown in SEQ ID NO:71, H-CDR2 has the amino acid sequence shown in SEQ ID NO:72 and H-CDR3 has the amino acid sequence shown in SEQ ID NO: 73.

3. The antibody or antigen-binding fragment according to claim 1, **characterized in that** the light chain variable region comprises complementarity determining regions: L-CDR1, L-CDR2 and L-CDR3; wherein,
(1) L-CDR1 has the amino acid sequence shown in SEQ ID NO:4, L-CDR2 has the amino acid sequence shown in SEQ ID NO:5 and L-CDR3 has the amino acid sequence shown in SEQ ID NO:6; or
(2) L-CDR1 has the amino acid sequence shown in SEQ ID NO:10, L-CDR2 has the amino acid sequence shown in SEQ ID NO:11 and L-CDR3 has the amino acid sequence shown in SEQ ID NO:12; or
(3) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:17; or
(4) L-CDR1 has the amino acid sequence shown in SEQ ID NO:4, L-CDR2 has the amino acid sequence shown in SEQ ID NO:16 and L-CDR3 has the amino acid sequence shown in SEQ ID NO:21; or
(5) L-CDR1 has the amino acid sequence shown in SEQ ID NO:25, L-CDR2 has the amino acid sequence shown in SEQ ID NO:26 and L-CDR3 has the amino acid sequence shown in SEQ ID NO:27; or
(6) L-CDR1 has the amino acid sequence shown in SEQ ID NO:31, L-CDR2 has the amino acid sequence shown in SEQ ID NO:32 and L-CDR3 has the amino acid sequence shown in SEQ ID NO:33; or
(7) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:39; or
(8) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:41; or
(9) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:45; or
(10) L-CDR1 has the amino acid sequence shown in SEQ ID NO:49, L-CDR2 has the amino acid sequence shown in SEQ ID NO:50 and L-CDR3 has the amino acid sequence shown in SEQ ID NO:51; or
(11) L-CDR1 has the amino acid sequence shown in SEQ ID NO:37, L-CDR2 has the amino acid sequence shown in SEQ ID NO:38 and L-CDR3 has the amino acid sequence shown in SEQ ID NO:55; or
(12) L-CDR1 has the amino acid sequence shown in SEQ ID NO:59, L-CDR2 has the amino acid sequence shown in SEQ ID NO:60 and L-CDR3 has the amino acid sequence shown in SEQ ID NO:51; or
(13) L-CDR1 has the amino acid sequence shown in SEQ ID NO:63, L-CDR2 has the amino acid sequence shown in SEQ ID NO:64 and L-CDR3 has the amino acid sequence shown in SEQ ID NO:65; or
(14) L-CDR1 has the amino acid sequence shown in SEQ ID NO:37, L-CDR2 has the amino acid sequence shown in SEQ ID NO:67 and L-CDR3 has the amino acid sequence shown in SEQ ID NO:41; or
(15) L-CDR1 has the amino acid sequence shown in SEQ ID NO:68, L-CDR2 has the amino acid sequence shown in SEQ ID NO:69 and L-CDR3 has the amino acid sequence shown in SEQ ID NO:70; or
(16) L-CDR1 has the amino acid sequence shown in SEQ ID NO:74, L-CDR2 has the amino acid sequence shown in SEQ ID NO:75 and L-CDR3 has the amino acid sequence shown in SEQ ID NO: 76.

4. The antibody or antigen-binding fragment according to any one of claims 1-3, **characterized in that** the antibody comprises at least one of a heavy chain framework region sequence and a light chain framework region sequence, at least a part of at least one of the heavy chain framework region sequence and the light chain framework region sequence is derived from at least one of a murine antibody, a human antibody, a primate antibody or a mutant thereof.

5. The antibody or antigen-binding fragment according to any one of claims 1-3, **characterized in that** the antibody heavy chain variable region comprises an amino acid sequence having at least 85%, at least 90%, at least 95%, or at least 99% sequence identity to any one of SEQ ID NO:77-92.

6. The antibody or antigen-binding fragment according to any one of claims 1-4, **characterized in that** the antibody light chain variable region comprises an amino acid sequence having at least 85%, at least 90%, at least 95%, or at least 99% sequence identity to any one of SEQ ID NO:93-108.

7. The antibody or antigen-binding fragment according to any one of claims 1-6, **characterized in that** the antibody comprises at least one of a heavy chain constant region and a light chain constant region, at least a part of at least one of the heavy chain constant region and the light chain constant region is derived from at least one of a murine antibody, a human antibody, a primate antibody or a mutant thereof;
preferably, both the antibody heavy chain constant region and the antibody light chain constant region are derived from a human IgG antibody or a mutant thereof;
more preferably, the antibody heavy chain constant region is derived from a human IgG1 or IgG4 antibody or a mutant thereof;
more preferably, the antibody light chain constant region is derived from a human IgGκ or a IgGλ constant region.

8. The antibody or antigen-binding fragment according to any one of claims 1-7, **characterized in that** the antibody heavy chain comprises variable region and constant region; the heavy chain variable region comprises an amino acid sequence having at least 85%, at least 90%, at least 95%, or at least 99% sequence identity to any one of SEQ ID NO:77-92.

9. The antibody or antigen-binding fragment according to any one of claims 1-8, **characterized in that** the antibody light chain comprises variable region and constant region; the light chain variable region comprises an amino acid sequence having at least 85%, at least 90%, at least 95%, or at least 99% sequence identity to any one of SEQ ID NO:93-108.

10. The antibody or antigen-binding fragment according to any one of claims 1-9, **characterized in that** the antibody is monoclonal antibody.

11. The antibody or antigen-binding fragment according to any one of claims 1-10, **characterized in that** the antigen-binding fragment of the antibody is selected from: Fab, Fab'-SH, Fv and (Fab')2 fragments.

12. The antibody or antigen-binding fragment according to any one of claims 1-11, **characterized in that** the antibody is a monospecific antibody, a bispecific antibody, or a polyspecific antibody.

13. An isolated nucleic acid molecule, **characterized in that** the nucleic acid molecule comprises a nucleotide sequence encoding the antibody or antigen-binding fragment according to any one of claims 1-12.

14. An expression vector, **characterized in that** the expression vector carries the nucleic acid molecule of claim 13.

15. A host cell, **characterized in that** the host cell comprises the nucleic acid molecule of claim 13 or the expression vector according to claim 14.

16. A pharmaceutical composition, **characterized in that** the pharmaceutical composition comprises the antibody or antigen-binding fragment of any one of claims 1-12, the nucleic acid molecule of claim 13, the expression vector of claim 14 or the host cell of claim 15 and a pharmaceutically acceptable carrier.

17. Use of the antibody or antigen-binding fragment of any one of claims 1-12, the nucleic acid molecule of claim 13, the expression vector of claim 14, the host cell of claim 15 or the pharmaceutical composition of claim 16 in the preparation of a medicament, the medicament is used for treating a cell proliferative disease or delaying the progression of a cell proliferative disease.

18. The use according to claim 17, **characterized in that** the cell proliferative disease is cancer.

19. The use according to claim 18, **characterized in that** the cancer is Ly6G6D-positive cancer.

20. The use according to claim 19, **characterized in that** the Ly6G6D-positive cancer is colorectal cancer, head and neck cancer, ovarian cancer, cervical cancer, melanoma, gastric cancer, esophageal cancer, small intestine cancer, large intestine cancer or adenocarcinoma, etc.

21. An antibody-drug conjugate, **characterized in that** the antibody-drug conjugate comprises the antibody or antigen-binding fragment of any one of claims 1-12.

22. A kit for detecting Ly6G6D, **characterized in that** the kit comprises the antibody or antigen-binding fragment of any one of claims 1-12.
